# EUROPÄISCHE PATENTSCHRIFT

(11) **EP 2 558 465 B1**
(45) Veröffentlichungstag und Bekanntmachung des Hinweises auf die Patenterteilung: **17.12.2014**
(21) Anmeldenummer: 11715496.3
(22) Anmeldetag: 14.04.2011
(51) Int. Cl.: C07D 471/14, C07D 487/14, C07D 498/14, A61K 31/407, A61K 31/437, A61K 31/4985, A61K 31/5383, A61P 7/02

(54) **TRIZYKLISCHE PYRIDYL-VINYL-PYRROLE ALS PAR1-INHIBITOREN**
TRICYCLIC PYRIDYL-VINYL-PYROLES AS PAR1 INHIBITORS
PYRIDYL-VINYLE-PYRROLES TRICYCLIQUES COMME INHIBITEURS DE PAR1

(30) Priorität: 16.04.2010 EP 10305396
(43) Veröffentlichungstag der Anmeldung: 20.02.2013
(73) Patentinhaber: SANOFI, 75008 Paris (FR)
(72) Erfinder: SCHOENAFINGER, Karl, 65926 Frankfurt am Main (DE); STEINHAGEN, Henning, 65926 Frankfurt am Main (DE); SCHEIPER, Bodo, 65926 Frankfurt am Main (DE); HEINELT, Uwe, 65926 Frankfurt am Main (DE); WEHNER, Volkmar, 65926 Frankfurt am Main (DE); HERRMANN, Matthias, 65926 Frankfurt am Main (DE)
(74) Vertreter: Venne-Dunker, Sabine
(86) Internationale Anmeldenummer: PCT/EP2011/055965
(87) Internationale Veröffentlichungsnummer: WO 2011/128421

(56) Entgegenhaltungen:
- US-A1- 2007 149 518
- CHACKALAMANNIL S ET AL: "Thrombin receptor (PAR-1) antagonists as novel antithrombotic agents", EXPERT OPINION ON THERAPEUTIC PATENTS, INFORMA HEALTHCARE, GB LNKD- DOI:10.1517/13543776.16.4.493, Bd. 16, Nr. 4, 1. April 2006 (2006-04-01), Seiten 493-505, XP002415653, ISSN: 1354-3776

## Beschreibung

Die Erfindung betrifft neue Verbindungen der Formel I, 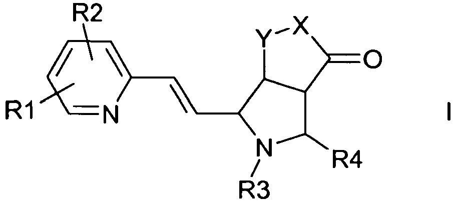 wobei R1, R2, R3, R4, X und Y die unten bezeichnete Bedeutung haben. Die Verbindungen der Formel I haben antithrombotische Aktivität und inhibieren insbesondere den Protease-aktivierten Rezeptor 1 (PAR1). Die Erfindung betrifft weiterhin ein Verfahren zur Herstellung der Verbindung der Formel I und deren Verwendung als Arzneimittel.

Der Protease-aktivierte Rezeptor 1 (PAR1) ist ein Thrombin Rezeptor, der zur Klasse der G-Protein-gekoppelten Rezeptoren (GPCR) gehört. Das Gen für PAR1 liegt auf Chromosom 5q13, besteht aus zwei Exonen und deckt eine Region von etwa 27 kb ab. PAR1 wird unter anderem in Endothelzellen, glatten Muskelzellen, Fibroblasten, Neuronen und humanen Blutplättchen exprimiert. Auf Blutplättchen ist PAR1 ein wichtiger Rezeptor der Signalübertragung welcher an der Initiation der Aggregation von Blutplättchen beteiligt ist. Die Aktivierung der PARs erfolgt über die proteolytische Abspaltung eines Teils des N-Terminus der PARs, wodurch eine neue N-terminale Sequenz freigelegt wird, die dann den Rezeptor aktiviert (M. D. Hollenberg et al., Pharmacol. Rev. 54:203-217, 2002).

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der vaskulären Konstriktion: Hierdurch wird der Blutverlust in das geschädigte Areal vermindert.
2. Die nächste Phase ist die der Plättchenadhäsion an das freigelegte Kollagen im Subendothel. Diese primäre Adhäsion an die Matrix aktiviert die Plättchen, die daraufhin verschiedene Aktivatoren sekretieren, die zur Verstärkung der Aktivierung führen. Diese Aktivatoren stimulieren zudem die weitere Rekrutierung neuer Plättchen zum Ort der Gefäßschädigung und fördern die Plättchenaggregation. Die Plättchen aggregieren an der Stelle des Gefäßwandschadens und bilden ein noch lockeres Plättchengerinnsel. Weiterhin führt die Aktivierung der Plättchen zur Präsentation von Phosphatidylserin und Phosphatidylinositol entlang der Zellmembranoberflächen. Die Exposition dieser Phospholipide ist zur Bindung und Aktivierung von Multienzymkomplexen der Blutgerinnungskaskade essentiell.
3. Das anfänglich noch lockere Plättchenaggregat wird durch Fibrin vernetzt. Wenn der Thrombus lediglich Plättchen und Fibrin enthält, handelt es sich um einen weißen Thrombus. Sind zusätzlich rote Blutkörperchen vorhanden, handelt es sich um einen roten Thrombus.
4. Nach Wundheilung wird der Thrombus durch die Einwirkung des Proteins Plasmin aufgelöst.

Zwei alternative Wege führen zur Bildung eines Fibringerinnsels, der intrinsische und der extrinsische Weg. Diese Wege werden durch unterschiedliche Mechanismen eingeleitet, in späterer Phase konvergieren sie jedoch zu einer gemeinsamen Wegstrecke der Gerinnungskaskade. Die Bildung eines roten Thrombus oder eines Gerinnsels auf dem Boden einer Gefäßwandabnormität ohne Wunde ist das Resultat des intrinsischen Weges. Die Fibringerinnselbildung als Antwort auf einen Gewebsschaden oder eine Verletzung ist das Resultat des extrinsischen Weges. Beide Wege beinhalten eine größere Anzahl von Proteinen, die als Gerinnungsfaktoren bekannt sind.

Der intrinsische Weg erfordert die Gerinnungsfaktoren VIII, IX, X, XI und XII sowie Präkallekrein, hochmolekulares Kininogen, Calciumionen und Phospholipide aus Plättchen. Jedes dieser Proteine führt zur Aktivierung des Faktors X. Der intrinsische Weg wird eingeleitet, wenn Präkallekrein, hochmolekulares Kininogen Faktor XI und XII an eine negativ geladene Oberfläche binden. Dieser Moment wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallekrein in Kallekrein, das wiederum den Faktor XII aktiviert. Faktor Xlla hydrolysiert weiteres Präkallekrein zu Kallekrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwesenheit von Ca²⁺-Ionen aktiviert der Faktor Xla den Faktor IX. Faktor IX ist ein Proenzym, das Vitamin-K abhängige, c-Karboxiglutamat (GLA)-Reste enthält. Die Serinproteaseaktivität kommt nach Bindung von Ca²⁺-Ionen an diese GLA-Reste zum Tragen. Mehrere der Serinproteasen der Blutgerinnungskaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Proteasekomplexes aus von Ca²⁺-Ionen und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Proteasekomplexes möglich. Faktor VIII hat in diesem Vorgang die Funktion eines Rezeptors für die Faktoren IXa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor Villa schließlich durch Thrombin weiter gespalten und inaktiviert. Diese duale Aktivität des Thrombins in Bezug zum Faktor VIII führt zu einer Selbstbegrenzung der Proteasekomplexbildung und damit zu einer Eingrenzung der Blutgerinnung.

Bei der Aktivierung von humanen Blutplättchen durch Thrombin spielen PAR1 und PAR4 eine zentrale Rolle; die Aktivierung dieser Rezeptoren führt in Blutplättchen zu morphologischen Veränderungen, Freisetzung von ADP und Aggregation der Blutplättchen (S.Brass, Nature 413:26-27, 2001).

Inhibitoren von PAR1 werden beispielsweise in EP 1391451, EP 1391452, US 6063847, US 2004/152736, US 2004/176418, US 2004/192753, US 2005/267155, US 2006/063847, US 2006/079684, US 2007/149518, US 2007/232635, US 6326380, WO 99/26943, WO 01/96330, WO 03/089428, WO 2006/076564, WO 2006/105217 und WO 2008/042422 beschrieben.

Es wurde gefunden, dass die Verbindungen der Formel I eine hohe spezifische Inhibierung des Protease-aktivierten Rezeptors 1 zeigen. Die Verbindungen der Formel I eignen sich daher für die prophylaktische als auch für die therapeutische Anwendung am Menschen, die an Erkrankungen leiden, die mit Thrombosen, Embolien, Hyperkoagulabilität oder fibrotischen Veränderungen einhergehen. Beispiele solcher Erkrankungen sind Thrombose, tiefe Venen-thrombose, Lungenembolien, Gehirninfarkt, Herzinfarkt, Bluthochdruck, entzündliche Erkrankungen, Rheuma, Asthma, Glomerulonephritis oder Osteoporose. Die Verbindungen der Formel I können zur Sekundär-Prävention eingesetzt werden und eignen sich sowohl für eine akute als auch für eine Langzeittherapie. Die Verbindungen der Formel I sind auch in Kombination mit Wirkstoffen einsetzbar, die über andere antithrombotische Prinzipien als PAR1 wirken.

Die Erfindung betrifft daher eine Verbindung der Formel I, 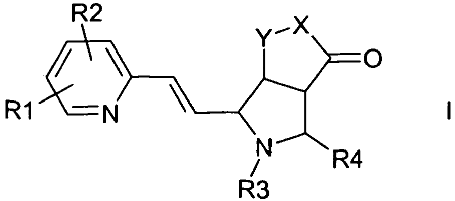 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei

R1 und R2 gleich oder verschieden sind und unabhängig voneinander für Wasserstoff, -(C₁-C₆)-Alkyl, -O-(C₁-C₆)-Alkyl, Aryl, Halogen oder Hetaryl stehen, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂, -NH₂, -OH, -O-CF₃, -S-CF₃ oder -CF₃ substituiert sind, oder
R1 und R2 zusammen mit den Ringatomen, an die sie jeweils gebunden sind, einen 5-gliedrigen bis 6-gliedrigen Ring bilden, wobei der Ring nur aus Kohlenstoffatomen besteht oder 1, 2 oder 3 dieser Atome durch N-, O- oder S-Atome ersetzt sind, wobei der Ring unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂, -NH₂, -OH, -O-CF₃, -S-CF₃ oder -CF₃ substituiert ist;

R3 und R4 zusammen mit den Ringatomen, an die sie jeweils gebunden sind, einen gesättigten oder ungesättigten 5-gliedrigen bis 7-gliedrigen Ring bilden, wobei der Ring nur aus Kohlenstoffatomen besteht oder 1 oder 2 dieser Atome durch O, S, SO, SO₂ oder N-R5 ersetzt sind, wobei der Ring unsubstituiert oder einfach, zweifach oder dreifach unabhängig voneinander durch F, Cl, -OH, -NH-R7, -(C₁-C₄)-Alkyl, -O-(C₁-C₄)-Alkyl, -C(O)-O-(C₁-C₄)-Alkyl, Aryl oder Hetaryl substituiert ist;
X für N-R6 oder O steht;
Y für C(O), CH₂, CH-CH₃ oder C(CH₃)₂ steht;
R5 für Wasserstoff, -C(O)-(C₁-C₄)-Alkyl, -C(O)-O-(C₁-C₄)-Alkyl, -(C₁-C₆)-Alkyl, -(C₂-C₄)-Alkyl-O-(C₁-C₄)-Alkyl, -(C₂-C₄)-Alkyl-OH, Aryl oder Hetaryl steht,
R7 für Wasserstoff, -C(O)-(C₁-C₄)-Alkyl, -C(O)-O-(C₁-C₄)-Alkyl, -(C₁-C₆)-Alkyl, -(C₂-C₄)-Alkyl-O-(C₁-C₄)-Alkyl, -(C₂-C₄)-Alkyl-OH, Aryl oder Hetaryl steht;
R6 für Wasserstoff, -(C₁-C₆)-Alkyl, -(C₃-C₇)-Cycloalkyl, -(C₁-C₄)-Alkylen-Aryl oder C(O)-O-(C₁-C₄)-Alkyl steht;
wobei unter dem Begriff "Hetaryl" Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff und Schwefel enthalten.

Die Erfindung betrifft weiterhin eine Verbindung der Formel I, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₆)-Alkyl, -O-(C₁-C₆)-Alkyl, Aryl, Halogen oder Hetaryl stehen, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂ oder -CF₃ substituiert sind, oder
R1 und R2 zusammen mit den Ringatomen, an die sie jeweils gebunden sind, einen Ring bilden, wobei das bicyclische Ringsystem, das aus diesem Ring und dem R1 und R2 tragenden Pyridinring besteht, ausgewählt ist aus der Gruppe Chinolin und Isochinolin;
R3 und R4 zusammen mit den Ringatomen, an die sie jeweils gebunden sind, einen gesättigten oder ungesättigten 5-gliedrigen bis 7-gliedrigen Ring bilden, wobei der Ring nur aus Kohlenstoffatomen besteht oder 1 oder 2 dieser Atome durch O, S, SO, SO₂ oder N-R5 ersetzt sind, ausgewählt aus der Gruppe Azepin, [1,2]Diazepin, [1,3]Diazepin, [1,4]Diazepin, Dihydroimidazolon, Imidazol, Imidazolin, Imidazolidin, Imidazolidinon, Isothiazol, Isothiazolidin, Isothiazolin, Isoxazol, Isoxazolin, Isoxazolidin, Morpholin, Oxathiazindioxid, [1,2]Oxazin, [1,3]Oxazin, [1,4]Oxazin, Oxazolon, Oxazol, Oxazolidin, Piperazin, Piperidin, Pyrazin, Pyrazol, Pyrazolin, Pyrazolidin, Pyridazin, Pyridin, Pyridinon, Pyridopyrazine, Pyridopyridine, Pyrimidin, Pyrrol, Pyrrolidin, Pyrrolidinon, Pyrrolin, 1,2,3,6-Tetrahydro-pyridin, [1,2]-Thiazin, [1,3]Thiazin, [1,4]Thiazin, [1,3]Thiazol, Thiazol oder Thiazolidin, wobei der Ring unsubstituiert oder einfach, zweifach oder dreifach unabhängig voneinander durch F, Cl, -OH, -(C₁-C₄)-Alkyl, -O-(C₁-C₄)-Alkyl, C(O)-O-(C₁-C₄)-Alkyl, Aryl oder Hetaryl substituiert ist;
X für N-R6 oder O steht;
Y für C(O), CH₂ oder CH-CH₃ steht;
R5 für Wasserstoff, C(O)-(C₁-C₄)-Alkyl, C(O)-O-(C₁-C₄)-Alkyl, -(C₁-C₆)-Alkyl, Aryl oder Hetaryl steht;
R6 für Wasserstoff, -(C₁-C₆)-Alkyl, -(C₃-C₇)-Cycloalkyl oder -(C₁-C₄)-Alkylen-Aryl steht;
wobei unter dem Begriff "Hetaryl" Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten.

Die Erfindung betrifft weiterhin eine Verbindung der Formel I, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₄)-Alkyl, -O-(C₁-C₄)-Alkyl, Br, F, Cl, Aryl oder Hetaryl stehen, wobei Hetaryl ausgewählt wird aus der Gruppe Acridinyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H,6H-1,5,2-Dithiazinyl, Furanyl, Furazanyl, Imidazolidinyl, Imidazolinyl, Imidazolyl, 1 H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pyridooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrolyl, Thienopyridinyl, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl und Xanthenyl, wobei Alkyl, Aryl und Hetaryl jeweils unsubstituiert oder einfach, zweifach oder dreifach unabhängig voneinander durch F, Cl, CN, -(C₁-C₄)-Alkyl, -O-(C₁-C₄)-Alkyl, -N((C₁-C₄)-Alkyl)₂ oder -CF₃ substituiert sind;
R3 und R4 zusammen mit den Ringatomen, an die sie jeweils gebunden sind, einen gesättigten oder ungesättigten 5-gliedrigen bis 7-gliedrigen Ring bilden, ausgewählt aus der Gruppe Azepin, [1,2]Diazepin, [1,3]Diazepin, [1,4]Diazepin, Dihydroimidazolon, Imidazol, Imidazolin, Imidazolidin, Imidazolidinon, Isothiazol, Isothiazolidin, Isothiazolin, Isoxazol, Isoxazolin, Isoxazolidin, Morpholin, Oxathiazindioxid, [1,2]Oxazin, [1,3]Oxazin, [1,4]Oxazin, Oxazolon, Oxazol, Oxazolidin, Piperazin, Piperidin, Pyrazin, Pyrazol, Pyrazolin, Pyrazolidin, Pyridazin, Pyridin, Pyridinon, Pyrimidin, Pyrrol, Pyrrolidin, Pyrrolidinon, Pyrrolin, 1,2,3,6-Tetrahydropyridin, [1,2]Thiazin, [1,3]Thiazin, [1,4]Thiazin, [1,3]Thiazol, Thiazol und Thiazolidin, wobei der Ring unsubstituiert oder einfach, zweifach oder dreifach unabhängig voneinander durch F, Cl, -OH, -(C₁-C₄)-Alkyl, C(O)-O-(C₁-C₄)-Alkyl, Aryl oder Hetaryl substituiert ist;
X für N-R6 oder O steht;
Y für C(O), CH₂ oder CH-CH₃ steht;
R6 für Wasserstoff, -(C₁-C₆)-Alkyl, -(C₃-C₆)-Cycloalkyl oder -(C₁-C₄)-Alkylen-Aryl steht.

Die Erfindung betrifft weiterhin eine Verbindung der Formel I, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₄)-Alkyl, Br, Thienyl oder Phenyl stehen, wobei Thienyl und Phenyl jeweils unsubstituiert oder einfach oder zweifach unabhängig voneinander durch F, Cl, -O-Methyl oder -CF₃ substituiert sind;
R3 und R4 zusammen mit den Ringatomen, an die sie jeweils gebunden sind, einen gesättigten oder ungesättigten 5-gliedrigen bis 6-gliedrigen Ring bilden, ausgewählt aus der Gruppe Azepin, [1,2]Diazepin, [1,3]Diazepin, [1,4]Diazepin, Dihydroimidazolon, Imidazol, Imidazolin, Imidazolidin, Imidazolidinon, Isothiazol, Isothiazolidin, Isothiazolin, Isoxazol, Isoxazolin, Isoxazolidin, Morpholin, Oxathiazindioxid, [1,2]Oxazin, [1,3]Oxazin, [1,4]Oxazin, Oxazolon, Oxazol, Oxazolidin, Piperazin, Piperidin, Pyrazin, Pyrazol, Pyrazolin, Pyrazolidin, Pyridazin, Pyridin, Pyridinon, Pyrimidin, Pyrrol, Pyrrolidin, Pyrrolidinon, Pyrrolin, 1,2,3,6-Tetrahydropyridin, [1,2]Thiazin, [1,3]Thiazin, [1,4]Thiazin, [1,3]Thiazol, Thiazol und Thiazolidin, wobei der Ring unsubstituiert oder einfach, zweifach oder dreifach unabhängig voneinander durch F, -OH, C(O)-O-(C₁-C₄)-Alkyl, Phenyl oder Hetaryl substituiert ist;
X für N-R6 oder O steht;
Y für C(O), CH₂ oder CH-CH₃ steht;

R6 für Wasserstoff, -(C₁-C₆)-Alkyl, -(C₃-C₆)-Cycloalkyl oder -(C₁-C₄)-Alkylen-Phenyl steht.

Die Erfindung betrifft weiterhin eine Verbindung der Formel I, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei

R1 und R2 gleich oder verschieden sind und unabhängig voneinander für Wasserstoff, -(C₁-C₄)-Alkyl, Br, Thienyl oder Phenyl stehen, wobei Thienyl und Phenyl jeweils unsubstituiert oder einfach oder zweifach unabhängig voneinander durch F, Cl, -O-Methyl oder -CF₃ substituiert sind;
R3 und R4 zusammen mit den Ringatomen, an die sie jeweils gebunden sind, einen gesättigten oder ungesättigten 5-gliedrigen bis 6-gliedrigen Ring bilden, ausgewählt aus der Gruppe Morpholin, Piperazin, Piperidin, Pyrrolidin, 1,2,3,6-Tetrahydropyridin und [1,3]Thiazol, wobei der Ring unsubstituiert oder einfach, zweifach oder dreifach unabhängig voneinander durch F, -OH, Phenyl oder C(O)-O-(C₁-C₄)-Alkyl substituiert ist;
X für N-R6 steht;
Y für C(O), CH₂ oder CH-CH₃ steht;
R6 für Wasserstoff, -(C₁-C₆)-Alkyl, Cyclopropyl oder -(C₁-C₄)-Alkylen-Phenyl steht.

Die Erfindung betrifft weiterhin eine Verbindung der Formel I, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, ausgewählt aus folgenden Verbindungen:
4-{((E)-2-[5-(3-Fluor-phenyl)-pyridin-2-yl]-vinyl}-2-methyl-octahydro-pyrrolo[3,4-a]indolizin-1,3-dion,
4-{((E)-2-[5-(3-Fluor-phenyl)-pyridin-2-yl]-vinyl}-2-methyl-octahydro-pyrrolo[3,4-a]indolizin-1,3-dion,
4-[((E)-2-(5-Brom-pyridin-2-yl)-vinyl]-2-methyl-octahydro-pyrrolo[3,4-a]indolizin-1,3-dion,
7-Hydroxy-2-methyl-4-[(E)-2-(6-methyl-pyridin-2-yl)-vinyl]-hexahydro-pyrrolo[3,4-a]-pyrrolizin-1,3-dion,
2-Methyl-4-[(E)-2-(6-methyl-pyridin-2-yl)-vinyl]-octahydro-pyrrolo[3,4-a]-indolizin-1,3-dion,
2-Methyl-4-[(E)-2-(6-methyl-pyridin-2-yl)-vinyl]-hexahydro-pyrrolo[3,4-a]-pyrrolizin-1,3-dion,
(8-{((E)-2-[5-(3-Fluor-phenyl)-pyridin-2-yl]-vinyl}-2-methyl-1,3-dioxo-decahydro-2,5,7a-triazacyclopenta[a]inden-5-carbonsäure-tert-butylester,
(S)-4-{(E)-2-[5-(3-Fluor-phenyl)-pyridin-2-yl]-vinyl}-7-hydroxy-2-methyl-hexahydro-pyrrolo[3,4-a]pyrrolizin-1,3-dion,
(R)-4-{(E)-2-[5-(3-Fluor-phenyl)-pyridin-2-yl]-vinyl}-7-hydroxy-2-methyl-hexahydro-pyrrolo[3,4-a]pyrrolizin-1,3-dion,
6-{((E)-2-[5-(3-Fluor-phenyl)-pyridin-2-yl]-vinyl}-8-methyl-hexahydro-pyrrolo[3',4':3,4]pyrrolo[2,1-c][1,4]oxazin-7,9-dion,
2-Ethyl-4-{(E)-2-[5-(3-fluor-phenyl)-pyridin-2-yl]-vinyl}-octahydro-pyrrolo[3,4-a]indolizin-1,3-dion,
4-{((E)-2-[5-(3-Fluor-phenyl)-pyridin-2-yl]-vinyl}-2-methyl-hexahydro-pyrrolo[3,4-a]pyrrolizin-1,3-dion,
5-{((E)-2-[5-(3-Fluor-phenyl)-pyridin-2-yl]-vinyl}-7-methyl-tetrahydro-pyrrolo[3',4':3,4]pyrrolo[1,2-c]thiazol-6,8-dion,
2-Methyl-4-{(E)-2-[5-(3-trifluormethyl-phenyl)-pyridin-2-yl]-vinyl}-octahydro-pyrrolo[3,4-a]indolizin-1,3-dion,
2-Methyl-4-{(E)-2-[5-(3-trifluormethyl-phenyl)-pyridin-2-yl]-vinyl}-octahydro-pyrrolo[3,4-a]indolizin-1,3-dion,
4-{((E)-2-[5-(2-Methoxy-phenyl)-pyridin-2-yl]-vinyl}-2-methyl-octahydro-pyrrolo[3,4-a]indolizin-1,3-dion,
4-{((E)-2-[5-(2-Chlor-phenyl)-pyridin-2-yl]-vinyl}-2-methyl-octahydro-pyrrolo[3,4-a]indolizin-1,3-dion,
2-Methyl-4-[(E)-2-(5-thiophen-3-yl-pyridin-2-yl)-vinyl]-octahydro-pyrrolo[3,4-a]indolizin-1,3-dion,
4-{((E)-2-[5-(3-Fluor-phenyl)-pyridin-2-yl]-vinyl}-2-methyl-3a,4,6,9,9a,9b-hexahydro-pyrrolo[3,4-a]indolizin-1,3-dion,
2-Cyclopropyl-4-{(E)-2-[5-(3-fluor-phenyl)-pyridin-2-yl]-vinyl}-octahydro-pyrrolo[3,4-a]indolizin-1,3-dion, und
8-{((E)-2-[5-(3-Fluor-phenyl)-pyridin-2-yl]-vinyl}-2-methyl-octahydro-2,5,7a-triazacyclopenta[a]inden-1,3-dion.

Unter dem Begriff "Alkyl", "-(C₁-C₆)-Alkyl" und "-(C₁-C₄)-Alkyl" werden Kohlenwasserstoffreste verstanden, deren Kohlenstoffkette geradkettig oder verzweigt ist und die 1, 2, 3, 4, 5 oder 6 Kohlenstoffatome bzw. 1, 2, 3 oder 4 Kohlenstoffatome enthalten, beispielsweise Methyl, Ethyl, Propyl, Isopropyl, Butyl, Isobutyl, sec-Butyl, tert-Butyl, Pentyl, Isopentyl, Neopentyl, 1-Ethylpropyl, Hexyl, 2,3-Dimethylbutyl oder Neohexyl. Bevorzugt enthält -(C₁-C₆)-Alkyl 1 bis 4 Kohlenstoffatome.

Unter dem Begriff "-O-Alkyl", "-O-(C₁-C₆)-Alkyl" und "-O-(C₁-C₄)-Alkyl" werden Alkoxyreste verstanden, deren Kohlenstoffkette geradkettig oder verzweigt ist und die 1, 2, 3, 4, 5 oder 6 Kohlenstoffatome bzw. 1, 2, 3 oder 4 Kohlenstoffatome enthalten, wie Methoxy, Ethoxy, Propoxy, Isopropoxy, Butoxy, Isobutoxy, tert-Butoxy, 1-Pentoxy, 2-Pentoxy, 3-Pentoxy, 1-Hexoxy, 2-Hexoxy oder 3-Hexoxy. Bevorzugt enthält -O-(C₁-C₆)-Alkyl 1 bis 4 Kohlenstoffatome.

Unter dem Begriff "-(C₃-C₇)-Cycloalkyl" werden 3-gliedrige bis 7-gliedrige Monocyclen verstanden wie Cyclopropyl, Cyclobutyl, Cyclopentyl, Cyclohexyl oder Cycloheptyl, unter dem Begriff "-(C₃-C₆)-Cycloalkyl" 3-gliedrige bis 6-gliedrige Monocyclen wie Cyclopropyl, Cyclobutyl, Cyclopentyl oder Cyclohexyl.

Unter dem Begriff "Aryl" werden aromatische Kohlenstoffreste verstanden mit 6 bis 14 Kohlenstoffatomen im Ring. Arylreste sind beispielsweise Phenyl, Naphthyl, zum Beispiel 1-Naphthyl, 2-Naphthyl, Anthryl oder Fluorenyl. Phenylreste und Naphthylreste, insbesondere Phenylreste, sind bevorzugte Arylreste.

Unter dem Begriff "Hetaryl" werden Ringsysteme verstanden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H,6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrolyl, Thienopyridinyl, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl oder Xanthenyl.

Unter dem Begriff "R3 und R4 bilden zusammen mit den Ringatomen, an die sie jeweils gebunden sind, einen gesättigten oder ungesättigten 5-gliedrigen bis 7-gliedrigen Ring, wobei der Ring nur aus Kohlenstoffatomen besteht oder 1 oder 2 dieser Atome durch O, S, SO, SO₂ oder N-R5 ersetzt sind" werden Ringsysteme verstanden wie Azepin, [1,2]Diazepin, [1,3]Diazepin, [1,4]Diazepin, Dihydroimidazolon, Imidazol, Imidazolin, Imidazolidin, Imidazolidinon, Isothiazol, Isothiazolidin, Isothiazolin, Isoxazol, Isoxazolin, Isoxazolidin, Morpholin, Oxathiazindioxid, [1,2]Oxazin, [1,3]Oxazin, [1,4]Oxazin, Oxazolon, Oxazol, Oxazolidin, Piperazin, Piperidin, Pyrazin, Pyrazol, Pyrazolin, Pyrazolidin, Pyridazin, Pyrrolidinon, Pyrrolin, 1,2,3,6-Tetrahydropyridin, [1,2]Thiazin, [1,3]Thiazin, [1,4]Thiazin, [1,3]Thiazol, Thiazol, Thiazolin oder Thiazolidin.

Unter dem Begriff "R1 und R2 bilden zusammen mit den Ringatomen, an die sie jeweils gebunden sind, einen 5-gliedrigen bis 6-gliedrigen Ring, wobei der Ring nur aus Kohlenstoffatomen besteht oder 1, 2 oder 3 dieser Atome durch N-, O- oder S-Atome ersetzt sind" werden beispielsweise die folgenden bicyclischen Ringsysteme, die aus diesem Ring und dem R1 und R2 tragenden Pyridinring bestehen, verstanden: Chinolin, 6,7-Dihydro-5H-[1]pyrindin, [1,3]Dioxolo[4,5-b]pyridin, Furo[3,2-b]pyridin, Isochinolin, [1,7]Naphthyridin, Pyridopyrazine, Pyrido[2,3-c]pyridazin, Pyridopyridine, Pyrido[2,3-d]pyrimidin, 5H-[1]Pyrindin, 1 H-Pyrrolo[2,3-b]pyridin, 5,6,7,8-Tetrahydrochinolin oder Thieno[3,2-b]pyridin.

Unter dem Begriff "Halogen" wird Fluor, Chlor, Brom oder Iod verstanden, bevorzugt sind Fluor, Chlor oder Brom, insbesondere Fluor oder Chlor.

Unter dem Begriff "X für O steht", das heißt "X für Sauerstoff oder ein Sauerstoffatom steht", wird der folgende Teilring der Formel I verstanden:

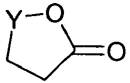

Unter dem Begriff "X für N-R6 steht" wird der folgende Teilring der Formel I verstanden:

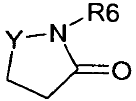

Unter dem Begriff "Y für C(O) steht" wird der folgende Teilring der Formel I verstanden:

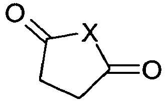

Unter dem Begriff "Y für CH₂ steht" wird der folgende Teilring der Formel I verstanden:

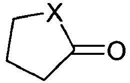

Unter dem Begriff "Y für CH-CH₃ steht" wird der folgende Teilring der Formel I verstanden:

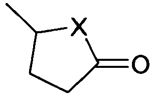

Unter dem Begriff "Y für C(CH₃)₂ steht" wird der folgende Teilring der Formel I verstanden:

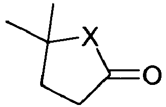

Funktionelle Gruppen in den Verbindungen der Formel I und den zu ihrer Herstellung verwendeten Intermediaten, beispielsweise Amino- oder Carboxylgruppen, können durch geeignete Schutzgruppen maskiert werden. Geeignete Schutzgruppen für Aminofunktionen sind beispielsweise die tert-Butoxycarbonyl-, die Benzyloxycarbonyl- oder die Phthaloylgruppe sowie die Trityl- oder Tosylschutzgruppe. Geeignete Schutzgruppen für die Carboxylfunktion sind beispielsweise Alkyl-, Aryl- oder Arylalkylester. Schutzgruppen können durch wohlbekannte oder hier beschriebene Techniken eingeführt und entfernt werden (siehe Greene, T.W., Wuts, P.G.M., Protective Groups in Organic Synthesis (1999), 3rd Ed., Wiley-Interscience, oder Kocienski, P. J., Protecting Groups (2004), 3rd Ed., Thieme). Der Begriff Schutzgruppe kann auch entsprechende polymergebundene Schutzgruppen umfassen.

In einer Ausführungsform der Erfindung sind R1 und R2 gleich oder verschieden und stehen unabhängig voneinander für Wasserstoff, -(C₁-C₆)-Alkyl, -O-(C₁-C₆)-Alkyl, Aryl, Halogen oder Hetaryl, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂, -NH₂, -OH, -O-CF₃, -S-CF₃ oder -CF₃ substituiert sind. In einer anderen Ausführungsform sind R1 und R2 gleich oder verschieden und stehen unabhängig voneinander für Wasserstoff, -(C₁-C₆)-Alkyl, -O-(C₁-C₆)-Alkyl, Aryl, Halogen oder Hetaryl, wobei Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂, -NH₂, -OH, -O-CF₃, -S-CF₃ oder -CF₃ substituiert sind. In einer anderen Ausführungsform sind R1 und R2 gleich oder verschieden und stehen unabhängig voneinander für Wasserstoff, -(C₁-C₆)-Alkyl, -O-(C₁-C₆)-Alkyl, Aryl, Halogen oder Hetaryl, wobei Aryl und Hetaryl jeweils unsubstituiert oder einfach, zweifach oder dreifach unabhängig voneinander durch F, Cl, Br, -(C₁-C₄)-Alkyl, -O-(C₁-C₄)-Alkyl, -O-CF₃ oder -CF₃ substituiert sind. In einer anderen Ausführungsform sind R1 und R2 gleich oder verschieden und stehen unabhängig voneinander für Wasserstoff, -(C₁-C₆)-Alkyl, Aryl, Halogen oder Hetaryl, wobei Aryl und Hetaryl jeweils unsubstituiert oder einfach, zweifach oder dreifach unabhängig voneinander durch F, Cl, Br, -(C₁-C₄)-Alkyl, -O-(C₁-C₄)-Alkyl, -O-CF₃ oder -CF₃ substituiert sind. In einer anderen Ausführungsform sind R1 und R2 gleich oder verschieden und stehen unabhängig voneinander für Wasserstoff, -(C₁-C₆)-Alkyl, Aryl, Halogen oder Hetaryl, wobei Aryl und Hetaryl jeweils unsubstituiert oder einfach, zweifach oder dreifach unabhängig voneinander durch F, Cl, Br oder -(C₁-C₄)-Alkyl substituiert sind.

In einer Ausführungsform der Erfindung steht eine der Gruppen R1 und R2 für Wasserstoff, -(C₁-C₆)-Alkyl, -O-(C₁-C₆)-Alkyl oder Halogen, in einer anderen Ausführungsform für Wasserstoff, -(C₁-C₆)-Alkyl oder Halogen, in einer anderen Ausführungsform für Wasserstoff oder Halogen, und die andere der Gruppen R1 und R2 steht in einer Ausführungsform für -(C₁-C₆)-Alkyl, -O-(C₁-C₆)-Alkyl, Aryl oder Hetaryl, in einer anderen Ausführungsform für -(C₁-C₆)-Alkyl, Aryl oder Hetaryl, in einer anderen Ausführungsform für Aryl oder Hetaryl, in einer anderen Ausführungsform für Phenyl, wobei das für die andere der Gruppen R1 und R2 stehende Alkyl, -O-Alkyl, Aryl, Phenyl oder Hetaryl in einer Ausführungsform jeweils unsubstituiert oder einfach, zweifach oder dreifach unabhängig voneinander durch F, Cl, Br, CN, -(C₁-C₄)-Alkyl, -O-(C₁-C₄)-Alkyl, -OH, -NH₂, -NH-(C₁-C₄)-Alkyl, -N((C₁-C₄)-Alkyl)₂, -OCF₃, -SCF₃ oder -CF₃ substituiert ist, in einer anderen Ausführungsform unsubstituiert oder einfach, zweifach oder dreifach unabhängig voneinander durch F, Cl, Br, -(C₁-C₄)-Alkyl, -O-(C₁-C₄)-Alkyl, -OCF₃ oder -CF₃ substituiert ist, in einer anderen Ausführungsform unsubstituiert oder einfach, zweifach oder dreifach unabhängig voneinander durch F, Cl, Br oder -(C₁-C₄)-Alkyl substituiert ist.

In einer Ausführungsform der Erfindung steht für R1 oder R2 stehendes Aryl für Phenyl, und in einer anderen Ausführungsform steht für R1 oder R2 stehendes Hetaryl für Thienyl, wobei Phenyl und Thienyl unsubstituiert oder wie angegeben substituiert sein können. In einer Ausführungsform der Erfindung sind substituierte Gruppen, die für R1 oder R2 stehen, durch einen oder zwei der angegebenen Substituenten substituiert, die gleich oder verschieden sein können.

In einer Ausführungsform der Erfindung ist der Ring, den R3 und R4 zusammen mit den Ringatomen, an die sie jeweils gebunden sind, bilden, gesättigt oder enthält eine oder zwei Doppelbindungen im Ring, in einer anderen Ausführungsform ist er gesättigt oder enthält eine Doppelbindung im Ring, in einer anderen Ausführungsform ist er gesättigt, in einer anderen Ausführungsform enthält er eine Doppelbindung im Ring. In einer Ausführungsform ist der Ring, den R3 und R4 zusammen mit den Ringatomen, an die sie jeweils gebunden sind, bilden, ein 5-gliedriger bis 6-gliedriger Ring, in einer anderen Ausführungsform ist er ein 5-gliedriger Ring, in einer anderen Ausführungsform ist er ein 6-gliedriger Ring. In einer Ausführungsform besteht der Ring, den R3 und R4 zusammen mit den Ringatomen, an die sie jeweils gebunden sind, bilden, abgesehen von dem die Gruppe R3 tragenden Stickstoffatom nur aus Kohlenstoffatomen oder es ist eines der Ring-Kohlenstoffatome durch O, S, SO, SO₂ oder N-R5 ersetzt, in einer anderen Ausführungsform besteht der Ring, den R3 und R4 zusammen mit den Ringatomen, an die sie jeweils gebunden sind, bilden, abgesehen von dem die Gruppe R3 tragenden Stickstoffatom nur aus Kohlenstoffatomen. Wenn in dem Ring, den R3 und R4 zusammen mit den Ringatomen, an die sie jeweils gebunden sind, bilden, einschließlich des die Gruppe R3 tragenden Stickstoffatoms mehr als ein Ring-Heteroatom vorhanden ist, befinden sich in einer Ausführungsform der Erfindung zwei Ring-Heteroatome nicht in benachbarten Ringpositionen. Wenn Ring-Kohlenstoffatome durch O, S, SO, SO₂ oder N-R5 ersetzt sind, sind sie in einer Ausführungsform ersetzt durch O, S oder N-R5, in einer anderen Ausführungsform durch O oder S, in einer anderen Ausführungsform durch O, in einer anderen Ausführungsform durch S, in einer anderen Ausführungsform durch S, SO oder SO₂, in einer anderen Ausführungsform durch N-R5. In einer Ausführungsform ist der Ring, den R3 und R4 zusammen mit den Ringatomen, an die sie jeweils gebunden sind, bilden, ein Pyrrolidinring, Piperidinring, Tetrahydropyridinring, Thiazolidinring, Oxazolidinring, Thiomorpholinring, Morpholinring oder Piperazinring, in einer anderen Ausführungsform ein Pyrrolidinring, Piperidinring, Thiazolidinring, Oxazolidinring, Thiomorpholinring, Morpholinring oder Piperazinring, in einer anderen Ausführungsform ein Pyrrolidinring, Piperidinring, Thiazolidinring, Morpholinring oder Piperazinring, in einer anderen Ausführungsform ein Pyrrolidinring, Piperidinring, Thiazolidinring oder Morpholinring, in einer anderen Ausführungsform ein Pyrrolidinring oder Piperidinring, die alle unsubstituiert oder wie angegeben substituiert sein können.

In einer Ausführungsform ist der Ring, den R3 und R4 zusammen mit den Ringatomen, an die sie jeweils gebunden sind, bilden, unsubstituiert oder einfach, zweifach oder dreifach unabhängig voneinander durch F, Cl, -OH, -NH-R7, -(C₁-C₄)-Alkyl, -O-(C₁-C₄)-Alkyl oder -C(O)-O-(C₁-C₄)-Alkyl substituiert, in einer anderen Ausführungsform ist er unsubstituiert oder einfach, zweifach oder dreifach unabhängig voneinander durch F, Cl, -OH, -(C₁-C₄)-Alkyl, -O-(C₁-C₄)-Alkyl oder -C(O)-O-(C₁-C₄)-Alkyl substituiert, in einer in einer anderen Ausführungsform ist er unsubstituiert oder einfach, zweifach oder dreifach unabhängig voneinander durch F, -OH, -(C₁-C₄)-Alkyl oder -O-(C₁-C₄)-Alkyl substituiert, in einer anderen Ausführungsform ist er unsubstituiert oder einfach, zweifach oder dreifach unabhängig voneinander durch F, -OH, -(C₁-C₄)-Alkyl, oder -C(O)-O-(C₁-C₄)-Alkyl substituiert.

In einer Ausführungsform der Erfindung steht X für N-R6, in einer anderen Ausführungsform für O.

In einer Ausführungsform der Erfindung steht Y für C(O), CH₂ oder -C(CH₃)₂, in einer anderen Ausführungsform für C(O) oder C(CH₃)₂, in einer anderen Ausführungsform für C(O), in einer anderen Ausführungsform für CH₂ oder C(CH₃)₂.

In einer Ausführungsform der Erfindung steht R5 für Wasserstoff, -C(O)-(C₁-C₄)-Alkyl, -C(O)-O-(C₁-C₄)-Alkyl, -(C₁-C₆)-Alkyl, -(C₂-C₄)-Alkyl-O-(C₁-C₄)-Alkyl oder -(C₂-C₄)-Alkyl-OH, in einer anderen Ausführungsform für Wasserstoff, -C(O)-(C₁-C₄)-Alkyl, -C(O)-O-(C₁-C₄)-Alkyl oder -(C₁-C₆)-Alkyl, in einer anderen Ausführungsform für Wasserstoff, -C(O)-O-(C₁-C₄)-Alkyl oder -(C₁-C₆)-Alkyl, in einer anderen Ausführungsform für Wasserstoff oder -C(O)-O-(C₁-C₄)-Alkyl.

In einer Ausführungsform der Erfindung steht R7 für Wasserstoff, -C(O)-(C₁-C₄)-Alkyl, -C(O)-O-(C₁-C₄)-Alkyl oder -(C₁-C₆)-Alkyl, in einer anderen Ausführungsform für Wasserstoff, -C(O)-(C₁-C₄)-Alkyl oder -C(O)-O-(C₁-C₄)-Alkyl, in einer anderen Ausführungsform für Wasserstoff oder -(C₁-C₆)-Alkyl.

In einer Ausführungsform der Erfindung steht R6 für Wasserstoff, -(C₁-C₆)-Alkyl, -(C₃-C₇)-Cycloalkyl oder C(O)-O-(C₁-C₄)-Alkyl, in einer anderen Ausführungsform für Wasserstoff, -(C₁-C₆)-Alkyl, -(C₃-C₇)-Cycloalkyl oder -(C₁-C₄)-Alkylen-Aryl, in einer anderen Ausführungsform für -(C₁-C₆)-Alkyl, -(C₃-C₇)-Cycloalkyl oder -(C₁-C₄)-Alkylen-Aryl, in einer anderen Ausführungsform für -(C₁-C₆)-Alkyl oder -(C₃-C₇)-Cycloalkyl, in einer anderen Ausführungsform für -(C₁-C₆)-Alkyl. In einer Ausführungsform ist für R6 stehendes -(C₃-C₇)-Cycloalkyl Cyclopropyl, Cyclobutyl, Cyclopentyl oder Cyclohexyl, in einer anderen Ausführungsform Cyclopropyl, Cyclobutyl oder Cyclopentyl, in einer anderen Ausführungsform Cyclopropyl oder Cyclobutyl, in einer anderen Ausführungsform Cyclopropyl.

Die Erfindung betrifft alle Kombinationen von Definitionen der Verbindungen der Formel I und einer oder mehrerer der beschriebenen Ausführungsformen, wie auch alle Kombinationen von Definitionen der Verbindungen der Formel I und einer oder mehrerer der beschriebenen Ausführungsformen und/oder einer oder mehrerer der beschriebenen spezifischen Bedeutungen, die eine Gruppe in den Verbindungen der Formel I haben kann.

Die erfindungsgemäßen Verbindungen können nach wohlbekannten Verfahren oder nach hier beschriebenen Verfahren hergestellt werden. Die Erfindung betrifft auch ein Verfahren zur Herstellung einer Verbindung der Formel I und/oder einer stereoisomeren oder tautomeren Form der Verbindung der Formel I und/oder eines physiologisch verträglichen Salzes der Verbindung der Formel I, nach dem die Verbindungen der Formel I, ihre stereoisomeren oder tautomeren Formen oder ihre physiologisch verträglichen Salze hergestellt werden können und das dadurch gekennzeichnet ist, dass man
a) eine Verbindung der Formel II, 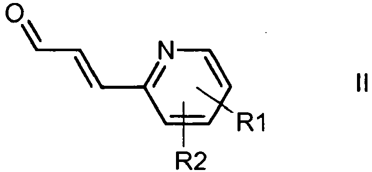 wobei die Reste R1 und R2 wie in Formel I definiert sind, mit einer Verbindung der Formel III und einer Verbindung der Formel IV, 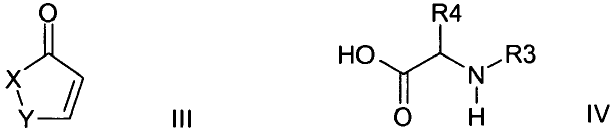 wobei die Reste X, Y, R3 und R4 wie in Formel I definiert sind, in Gegenwart eines Lösungsmittels bei 20°C bis 120°C zu einer Verbindung der Formel I umsetzt; oder
b) eine Verbindung der Formel V 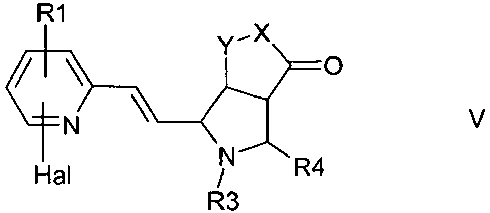 wobei die Reste X, Y, R1, R3 und R4 wie in Formel I definiert sind und Hal die Bedeutung Chlor, Brom, Iod oder Triflat (Trifuormethansulfonyloxy) hat, mit einer Verbindung der Formel R2-B(OH)₂ oder einem Derivat davon in Gegenwart einer Base und eines geeigneten Metallkatalysators in einem geeigneten Lösemittel oder Lösemittelgemisch zu einer Verbindung der Formel I umsetzt; oder
c) eine Verbindung der Formel I, worin X die Bedeutung NH hat, mit einem geeigneten Alkylierungsmittel in Gegenwart einer Base in einem geeigneten inerten Lösemittel bei Raumtemperatur oder bei erhöhter Temperatur zu einer Verbindung der Formel I umsetzt, worin X die Bedeutung N-R6 hat und R6 -(C₁-C₆)-Alkyl, -(C₃-C₇)-Cycloalkyl oder -(C₁-C₄)-Alkylen-Aryl bedeutet; oder
d) die nach den Verfahren a) bis c) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreiner Verbindungen wie zum Beispiel Aminosäuren, Trennung der somit erhaltenen Diastereomeren, und Abspaltung der chiralen Hilfsgruppen in die reinen Enantiomeren oder Diastereomeren auftrennt; oder
e) die nach den Verfahren a) bis d) hergestellte Verbindung der Formel I entweder in freier Form isoliert, oder aus physiologisch unverträglichen Salzen freisetzt, oder im Falle des Vorliegens von sauren oder basischen Gruppen in ein physiologisch verträgliches Salz umwandelt.

Die Verbindungen der Formel II können hergestellt werden durch Umsetzung eines Aldehyds der Formel VI mit einer Verbindung der Formel VII 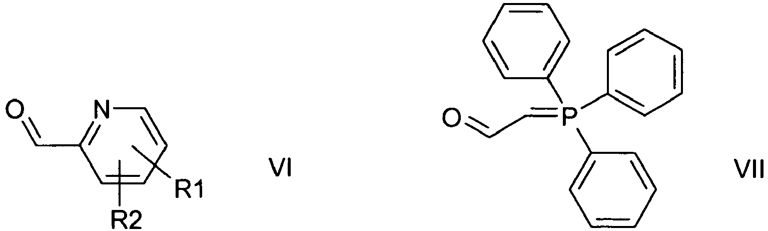 oder einem anderen geeigneten Phosphorylid-Reagenzien in einem geeigneten Lösemittel und ggf. bei erhöhter Temperatur. Aldehyde der Formel VI sind entweder kommerziell erhältlich oder lassen sich nach bekannten Verfahren herstellen. So kann man beispielsweise halogensubstituierte Pyridinaldehyde in Gegenwart von geeigneten Übergangsmetallkatalysatoren wie Palladium oder Nickel und deren Phosphankomplexen mit Borsäurederivaten umsetzen zu Alkyl-, Aryl- und Hetarylsubstituierten Derivaten der Formel VI.

Saure oder basische Verbindungen der Formel I können in Form ihrer Salze oder in freier Form vorliegen. Bevorzugt sind pharmakologisch verträgliche Salze, insbesondere pharmazeutisch akzeptable Salze, beispielsweise Alkali- oder Erdalkalimetallsalze oder Hydrochloride, Sulfate, Hemisulfate, Methylsulfonate, p-Toluolsulfonate, alle möglichen Phosphate sowie Salze der Aminosäuren, natürlicher Basen oder Carbonsäuren wie Lactate, Citrate, Tartrate, Acetate, Adipinate, Fumarate, Gluconate, Glutamate, Maleinate oder Palmoate. Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Enthalten Verbindungen der Formel I saure Funktionalität, so lassen sich mit basischen Reagenzien wie Hydroxiden, Carbonaten, Hydrogencarbonaten, Alkoholaten sowie Ammoniak oder organischen Basen, beispielsweise Trimethylamin oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze bilden. Basische Gruppen der Verbindungen der Formel I, bilden mit Säuren Säureadditionssalze. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzolsulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin- oder Trifluoressigsäure in Frage.

Die Verbindungen der Formel I können mehrere asymetrische Kohlenstoffatome enthalten und in der Form von Diastereomeren oder Enantiomeren oder Gemischen derselben auftreten. Sofern die Verbindung der Formel I als Gemisch von Diastereomeren oder Enantiomeren auftritt oder bei der gewählten Synthese als deren Gemisch anfällt, kann sie in die reinen Stereoisomeren getrennt werden, entweder durch Chromatographie an einem gegebenenfalls chiralen Trägermaterial, oder, sofern die racemische Verbindung der Formel I zur Salzbildung befähigt ist, kann auch eine fraktionierte Kristallisation der mit einer optisch aktiven Base oder Säure als Hilfsstoff gebildeten diastereomeren Salze durchgeführt werden. Als chirale Stationärphasen für die dünnschichtchromatographische oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L-Weinsäure, D- und L- Milchsäure sowie (+) und (-)-Mandelsäure in die reinen Enantiomeren überführen. Auch kann man chirale Verbindungen, die Alkohol- oder Amin-Funktionen enthalten, mit entsprechend aktivierten oder gegebenenfalls N-geschützten enantiomerenreinen Aminosäuren in die entsprechenden Ester oder Amide überführen, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäurerestes oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführten chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereoreine oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literaturbekannten Verfahren enantiomerenrein oder diastereomerenrein hergestellt. Das kann insbesondere bedeuten, dass in der Synthese der Grundgerüste entweder enantioselektive Verfahren zum Einsatz kommen, oder aber eine Enantiomerentrennung oder Diastereomerentrennung auf früher Synthesestufe und nicht erst auf der Stufe der Endprodukte durchgeführt wird. Ebenso kann eine Vereinfachung der Trennungen dadurch erreicht werden, dass zweistufig oder mehrstufig vorgegangen wird.

Die Erfindung betrifft auch Arzneimittel und pharmazeutische Zubereitungen, gekennzeichnet durch einen wirksamen Gehalt an mindestens einer Verbindung der Formel I und/oder eines physiologisch verträglichen Salzes der Verbindung der Formel I und/oder einer stereoisomeren oder tautomeren Form der Verbindung der Formel I, zusammen mit einem pharmazeutisch geeigneten und physiologisch verträglichen Trägerstoff, Zusatzstoff und/oder anderen Wirkstoffen und Hilfsstoffen. Die Erfindung betrifft weiterhin eine Verbindung der Formel I und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, zur Verwendung als Pharmazeutikum oder Wirkstoff in einem Arzneimittel.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung des Protease-aktivierten Rezeptors 1 (PAR1) behandelbar sind. So eignen sich die erfindungsgemäßen Verbindungen sowohl für eine prophylaktische als auch für eine therapeutische Anwendung am Menschen. Sie eignen sich sowohl für eine akute Behandlung als auch für eine chronische Behandlung in einer Langzeittherapie. Die Verbindungen der Formel I können eingesetzt werden in Patienten, die an Störungen des Wohlbefindens oder Krankheiten leiden, die mit Thrombosen, Embolien, Hyperkoagulabilität oder fibrotischen Veränderungen einhergehen. Dazu gehören der Myokardinfarkt, die Angina pectoris und alle anderen Formen des akuten Koronarsyndroms, den akuten Schlaganfall oder dessen Sekundärprevention, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen.

Weiterhin können die Verbindungen der Formel I eingesetzt werden bei allen Eingriffen, die zu einem Kontakt des Blutes mit Fremdoberflächen führen wie bei Dialysepatienten und Patienten mit Verweilkathetern. Verbindungen der Formel I können eingesetzt werden, um die Thrombosegefahr nach chirurgischen Eingriffen wie bei Knie- und Hüftgelenksoperationen zu reduzieren. Verbindungen der Formel I eignen für die Behandlung von Patienten mit disseminierter intravaskulärer Koagulation, Sepsis und anderen intravaskulären Ereignissen, die mit einer Entzündung einhergehen.

Weiterhin eignen sich Verbindungen der Formel I für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. Verbindungen der Formel I eignen sich zur Verlangsamung oder Verhinderung solcher Prozesse.

Weitere Indikationen für den Einsatz der Verbindungen der Formel I sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formel I eignen sich auch zur Verhinderung und/oder Behandlung von Narbenbildung.

Die Applikation der erfindungsgemäßen Arzneimittel kann zum Beispiel durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung von Stents mit Verbindungen der Formel I und anderen Oberflächen, die im Körper mit Blut in Kontakt kommen, ist möglich.

Die Erfindung betrifft auch ein Verfahren zur Herstellung eines Arzneimittels, das dadurch gekennzeichnet ist, dass man mindestens eine Verbindung der Formel I mit einem pharmazeutisch geeigneten und physiologisch verträglichen Träger und gegebenenfalls weiteren geeigneten Wirkstoffen, Zusatzstoffen oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Sprengmittel, Bindemittel, Überzugsmittel, Quellungsmittel, Gleitmittel oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumenöl, Erdnussöl oder Sesamöl, Polyethylenglykol und Lösungsmittel, wie etwa steriles Wasser und einwertige oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt etwa 50 mg bis 300 mg und bei Injektionslösungen in Ampullenform bis zu etwa 300 mg, bevorzugt etwa 10 mg bis 100 mg, betragen.

Für die Behandlung eines erwachsenen, etwa 70 kg schweren Patienten sind je nach Wirksamkeit der Verbindung der Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Verbindungen der Formel I können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden. Als Plättchenaggregationshemmer kommen dabei Cyclooxygenase 1-Inhibitoren wie Aspirin, irreversible P2Y₁₂-Antagonisten wie Clopidogrel oder Prasugrel, reversible P2Y₁₂-Antagonisten wie Cangrelor oder AZD6140 und Thromboxan A₂/Prostaglandin H₂-Antagonisten wie Terutroban in Frage. Additive Effekte der PAR1-Blockade in Kombination mit P2Y₁₂-Blockade konnten beispielsweise gezeigt werden (M. Chintala et al., Eur. Heart J. 28 (Abstract Supplement 1): 188, 2007).

### Beispiele

Die hergestellten Verbindungen wurden in der Regel durch spektroskopische und chromatographische Daten, im speziellen Massenspektren (MS) und HPLC-Retentionszeiten (Rt; in min) charakterisiert, welche durch kombinierte, analytische HPLC/MS-Charakterisierung (LC/MS) erhalten wurden. In der MS-Charakterisierung ist, in der Regel die Massennummer (m/z) des Peaks des Molekülions (M, bzw. M⁺) oder eines verwandten Ions wie des Ions M+1 (bzw. M+1⁺; protoniertes Molekülion M+H⁺), welches sich abhängig von der Ionisierungsmethode die benutzt wurde gebildet hat, angegeben. Im allgemeinen wurde die Ionisationsmethode Elektrospray-Ionisation (ESI) benutzt. Folgende LC/MS-Methoden wurden benutzt:

### Methode D

Säule: YMC J'sphere ODS H80 20x2,1 mm 4 µm
Lösungsmittel: MeCN:H₂O + 0.05% TFA (Fluss 1 mL/min)
Gradient: von 4:96 (0 min) bis 95:5 (2 min) bis 95:5 (2,4 min) bis 96:4 (2,45 min)
Ionisation: ESI⁺

### Methode J

Säule: Luna C18 10x2 mm 3 µm
Lösungsmittel: MeCN + 0.05% TFA:H₂O + 0.05% TFA (Fluss 1,1 mL/min)
Gradient: 7:93 (0 min) bis 95:5 (1,2 min) bis 95:5 (1,4 min) bis 7:93 (1,45 min)
Ionisation: ESI⁺

Weiterhin erfolgte eine Charakterisierung der Verbindungen durch ¹H-NMR-Spektroskopie. Verwendete Abkürzungen sind entweder erläutert oder entsprechen den üblichen Konventionen. Das Abdampfen von Lösungsmitteln geschah in der Regel unter vermindertem Druck bei 35 °C bis 45 °C am Rotationsverdampfer und wird mit "vom Lösungsmittel befreit", "eingeengt", "abgedampft" oder "Lösungsmittel entfernt" umschrieben. Die Umsetzungen fanden in Standard-Reaktionsapparaturen wie Einhalskolben oder Mehrhalskolben statt, die wenn nicht anders beschrieben, dem Bedarf angemessen 5 ml bis 2000 ml Volumen fassten und je nach Anforderung mit Septum, Stopfen, Kühler, Rührer oder anderen Ausrüstungsgegenständen bestückt waren. Wenn nicht anders erwähnt, fanden alle Reaktionen unter Argon als Schutzgas statt und wurden mit Magnetrührern gerührt.

**Verwendete Abkürzungen:**

| | |
|---|---|
| Boc | tert-Butoxycarbonyl |
| DCM | Dichlormethan |
| DMSO | Dimethylsulfoxid |
| ges. | gesättigt |
| MeCN | Acetonitril |
| MeOH | Methanol |
| Rt | Retentionszeit |
| RT | Raumtemperatur (20 °C bis 25 °C) |
| TFA | Trifluoressigsäure |

Bei den Umsetzungen werden in der Regel mehrere Diastereomere gebildet, die sich als racemische Mischungen säulenchromatografisch trennen lassen. Wenn nicht angegeben ist eine Zuordnung dieser racemischen Mischungen zu bestimmten Konfigurationen noch nicht eindeutig erfolgt. Ebenfalls noch nicht erfolgt ist die absolute Zuordnung der reinen Diastereomeren, die aus den racemischen Mischungen durch chirale Säulenchromatografie erhalten werden. Bei Angabe einer definierten Stereochemie erfolgte die Zuordnung aus den Kopplungskonstanten der Wasserstoffatome im Pyrrolring durch NMR-spektroskopische Verfahren.

### Beispiel 1

### 5-(3-Fluor-phenyl)-pyridin-2-carbaldehyd

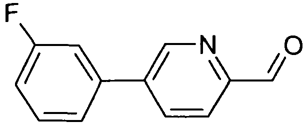

Die Mischung von 2 g 5-Brom-pyridin-2-carbaldehyd, 1,96g 3-Fluor-phenylboronsäure, 11,2 g K₂CO₃, 160 ml Toluol, 60 ml Wasser, 60 ml Ethanol und 0,93 mg Tetrakis(triphenylphosphin)palladium(0) wird unter Argon bei 100 °C 2 Stunden gerührt. Die Lösemittel werden abgedampft und der Rückstand in 100 ml Wasser dispergiert und das Produkt mit 2 Portionen von 30 ml Ethylacetat extrahiert. Die organische Phase wird mit 30 ml ges. Kochsalzlösung gewaschen, über Natriumsulfat getrocknet und eingeengt. Der resultierende Rückstand wird aus 28 ml Isopropanol umkristallisiert:
Ausbeute: 1,3 g, LC/MS (Methode D): m/z = 202 (M+1); Rt = 1,317 min

### Beispiel 2

### (E)-3-[5-(3-Fluor-phenyl)-pyridin-2-yl]-propenal

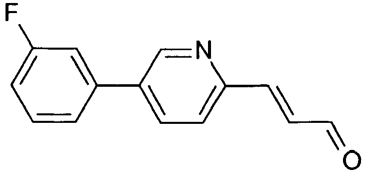

584 mg 5-(3-Fluor-phenyl)-pyridin-2-carbaldehyd und 883 mg (Triphenylphosphoranyliden)-acetaldehyd werden bei RT über Nacht gerührt. Die Lösemittel werden abgedampft und der Rückstand durch Säulen-Chromatografie (Kieselgel, MeOH:DCM = 99,5:0,5) gereinigt.
Ausbeute: 450 mg, LC/MS (Methode J): m/z = 228 (M+1); Rt = 0,887 min

### Beispiel 3

### (E)-3-(5-Brom-pyridin-2-yl)-propenal

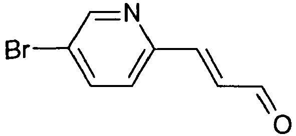

818 mg (Triphenylphosphoranyliden)-acetaldehyd und 500 mg 5-Brom-pyridin-2-carbaldehyd werden bei RT über Nacht gerührt. Die flüchtigen Anteile werden unter verminderten Druck entfernt und der Rückstand wird durch eine Säulenchromatografie (Kieselgel, MeOH:DCM = 99:1) gereinigt.
Ausbeute: 415 mg; LC/MS (Methode D): m/z = 213 (M+1); Rt = 1,121 min

### Beispiel 4

### 4-{(E)-2-[5-(3-Fluor-phenyl)-pyridin-2-yl]-vinyl}-2-methyl-octahydro-pyrrolo[3,4-a]indolizin-1,3-dion (2 racemische Mischungen)

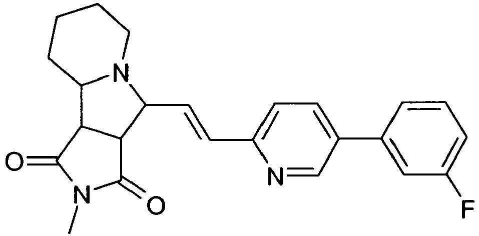

Die Mischung von 235 mg (E)-3-[5-(3-Fluor-phenyl)-pyridin-2-yl]-propenal, 133 mg D-Pipecolinsäure, 115 mg N-Methylmaleinimid und 30 ml MeCN werden 2 h zum Sieden erhitzt. Die flüchtigen Anteile werden unter verminderten Druck entfernt und der Rückstand durch Säulenchromatografie (Kieselgel, MeOH:DCM = 97:3) gereinigt. Auf diese Weise werden 2 reine Fraktionen isoliert.
Ausbeuten: Racemische Mischung 1: 275 mg; LC/MS (Methode D): m/z = 406 (M+1); Rt = 0,985 min
Racemische Mischung 2: 115 mg; LC/MS (Methode D): m/z = 406 (M+1); Rt = 0,994 min

### Beispiel 5

### 4-{(E)-2-[5-(3-Fluor-phenyl)-pyridin-2-yl]-vinyl}-2-methyl-octahydro-pyrrolo[3,4-a]indolizin-1,3-dion (2 Diastereomere)

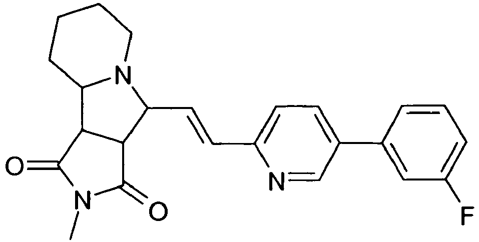

Die racemische Mischung 2 aus Beispiel 4 wird durch eine chirale Chromatografie (HPLC Säule: Chiralcel OJ-H/62, 250x4,6 mm, Eluent: MeOH + 0,1% Diethylamin, Flußrate: 1 ml/min, 30 °C) getrennt und liefert 2 reine Diastereomere von noch unbekannter absoluter Konfiguration.
Ausbeuten: Diastereomer 1: 43 mg; LCMS: (M+1): 406; Rt = 6,27 min
Diastereomer 2: 42,5 mg; LC/MS: (M+1): 406; Rt = 7,64 min

### Beispiel 6

### 4-[(E)-2-(5-Brom-pyridin-2-yl)-vinyl]-2-methyl-octahydro-pyrrolo[3,4-a]indolizin-1,3-dion (3 racemische Mischungen)

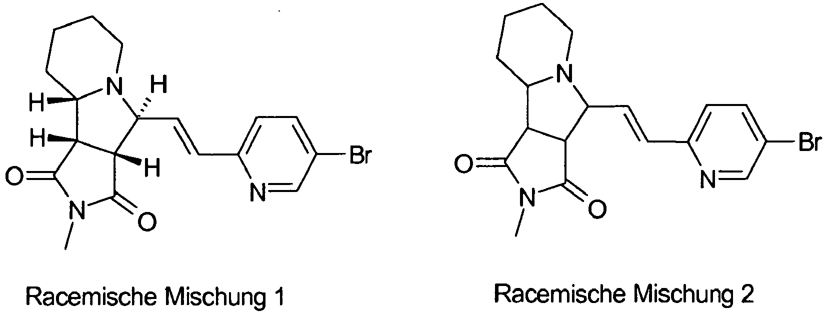

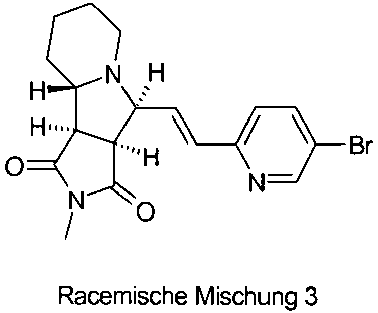

Die Mischung von 820 mg (E)-3-(5-Brom-pyridin-2-yl)-propenal, 500 mg D-Pipecolinsäure, 430 mg N-Methylmaleinimid und 20 ml MeCN wird 2 h am Rückfluss gekocht. Die Lösemittel werden abgezogen und der Rückstand durch Säulenchromatografie (Kieselgel, Ethylacetat:DCM = 3 : 1) gereinigt. Mit dieser Methode werden 3 reine Fraktionen erhalten.
Ausbeute: Racemische Mischung 1: 414 mg; LC/MS (Methode J): m/z = 391 (M+1); Rt = 0,598 min
Racemische Mischung 2: 162 mg; LC/MS (Methode J): m/z = 391 (M+1); Rt = 0,583 min
Racemische Mischung 3: 385 mg; LC/MS (Methode J): m/z = 391 (M+1); Rt = 0,600 min

### Beispiel 7

### 7-Hydroxy-2-methyl-4-[(E)-2-(6-methyl-pyridin-2-yl)-vinyl]-hexahydro-pyrrolo[3,4-a]pyrrolizin-1,3-dion (2 racemische Mischungen)

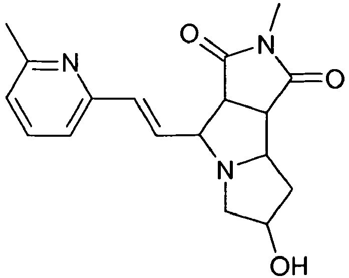

Die Titelverbindung wird in analoger Weise ausgehend von 100 mg (E)-3-(6-Methyl-pyridin-2-yl)-propenal, 75 mg N-Methylmaleinimid und 100 mg cis-4-Hydroxy-L-prolin hergestellt.
Ausbeuten: Racemische Mischung 1: 15 mg; LC/MS (Methode J): m/z = 328 (M+1); Rt = 0,106 min
Racemische Mischung 2: 173 mg; LC/MS (Methode J): m/z = 328 (M+1); Rt = 0,102 min

### Beispiel 8

### 2-Methyl-4-[(E)-2-(6-methyl-pyridin-2-yl)-vinyl]-octahydro-pyrrolo[3,4-a]-indolizin-1,3-dion (3 racemische Mischungen)

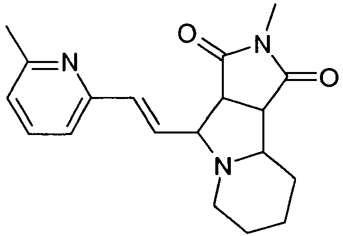

Die Titelverbindung wird in analoger Weise ausgehend von 100 mg (E)-3-(6-Methyl-pyridin-2-yl)-propenal, 76 mg N-Methylmaleinimid und 78 mg Pipecolinsäure erhalten. Der Rückstand wird durch Säulenchromatografie (Kieselgel, MeOH:DCM = 2 : 98) gereinigt.
Ausbeuten: Racemische Mischung 1: 27 mg; LC/MS (Methode J): m/z = 326 (M+1); Rt = 0,164 min
Racemische Mischung 2: 71 mg; LC/MS (Methode J): m/z = 326 (M+1); Rt = 0,155 min
Racemische Mischung 3: 44 mg; LC/MS (Methode J): m/z = 326 (M+1); Rt = 0,125 min

### Beispiel 9

### 2-Methyl-4-[(E)-2-(6-methyl-pyridin-2-yl)-vinyl]-hexahydro-pyrrolo[3,4-a]-pyrrolizin-1,3-dion (3 racemische Mischungen)

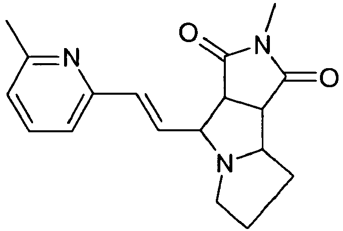

Die Titelverbindung wird in analoger Weise ausgehend von 100 mg (E)-3-(6-Methyl-pyridin-2-yl)-propenal, 76 mg N-Methylmaleinimid und 78 mg L-Prolin erhalten. Der Rückstand wurde durch Säulenchromatografie (Kieselgel, MeOH:DCM = 2 : 98) gereinigt.
Ausbeuten: Racemische Mischung 1: 38 mg, LC/MS (Methode J): m/z = 312 (M+1); Rt = 0,106 min
Racemische Mischung 2: 34 mg; LC/MS (Methode J): m/z = 312 (M+1); Rt = 0,114 min
Racemische Mischung 3: 66 mg; LC/MS (Methode J): m/z = 312 (M+1); Rt = 0,113 min

### Beispiel 10

### (8-{(E)-2-[5-(3-Fluor-phenyl)-pyridin-2-yl]-vinyl}-2-methyl-1,3-dioxo-decahydro-2,5,7a-triazacyclopenta[a]inden-5-carbonsäure-tert-butylester (2 racemische Mischungen)

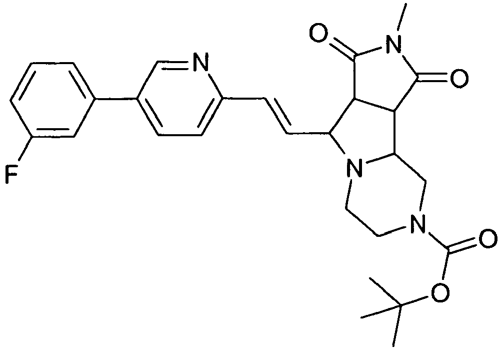

Die Mischung von 50 mg (E)-3-[5-(3-Fluor-phenyl)-pyridin-2-yl]-propenal, 50 mg 4-Boc-piperazin-2-carbonsäure, 24 mg N-Methylmaleinimid und 2 ml MeCN werden 2 h am Rückfluss erhitzt. Die flüchtigen Anteile werden unter verminderten Druck entfernt und der Rückstand durch Säulenchromatografie (Kieselgel, MeOH:DCM = 98:2) gereinigt. Auf diese Weise werden 2 reine Fraktionen isoliert.
Ausbeuten: Racemische Mischung 1: 11 mg; LC/MS (Methode J): m/z = 507 (M+1); Rt = 0,933 min
Racemische Mischung 2: 10 mg; LC/MS (Methode J): m/z = 507 (M+1); Rt = 0,879 min

### Beispiel 11

### (S)-4-{(E)-2-[5-(3-Fluor-phenyl)-pyridin-2-yl]-vinyl}-7-hydroxy-2-methyl-hexahydro-pyrrolo[3,4-a]pyrrolizin-1,3-dion (4 racemische Mischungen)

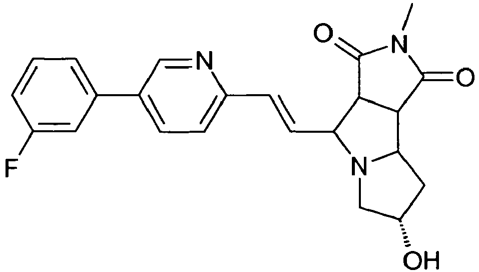

Die Mischung von 100 mg (E)-3-[5-(3-Fluor-phenyl)-pyridin-2-yl]-propenal, 64 mg (2S,4S)-4-Hydroxy-pyrrolidin-2-carbonsäure, 54 mg N-Methylmaleinimid und 4 ml MeCN werden 4 h zum Sieden erhitzt. Die flüchtigen Anteile werden unter verminderten Druck entfernt und der Rückstand durch Säulenchromatografie (Kieselgel, MeOH:DCM = 5:95) gereinigt. Auf diese Weise werden 4 reine Faktionen als racemische Mischungen isoliert.
Ausbeuten: Racemische Mischung 1: 47 mg; LC/MS (Methode J): m/z = 408 (M+1); Rt = 0,667 min
Racemische Mischung 2: 17 mg; LC/MS (Methode J): m/z = 408 (M+1); Rt = 0,664 min
Racemische Mischung 3: 18 mg; LC/MS (Methode J): m/z = 408 (M+1); Rt = 0,667 min
Racemische Mischung 4: 18 mg; LC/MS (Methode J): m/z = 408 (M+1); Rt = 0,673 min

### Beispiel 12

### (R)-4-{(E)-2-[5-(3-Fluor-phenyl)-pyridin-2-yl]-vinyl}-7-hydroxy-2-methyl-hexahydro-pyrrolo[3,4-a]pyrrolizin-1,3-dion (4 racemische Mischungen)

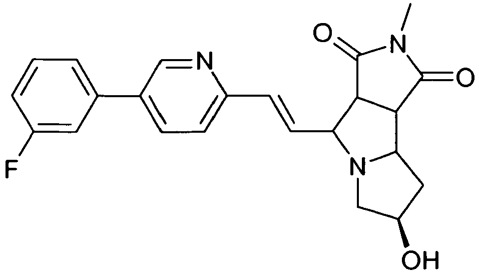

Die Titelverbindung wird in analoger Weise ausgehend von 100 mg (E)-3-[5-(3-Fluor-phenyl)-pyridin-2-yl]-propenal, 54 mg N-Methylmaleinimid und 64 mg (2S,4R)-4-Hydroxy-pyrrolidin-2-carbonsäure erhalten. Der Rückstand wird durch Säulenchromatografie (Kieselgel, MeOH:DCM = 5:95) gereinigt. Auf diese Weise werden 4 reine Faktionen als racemische Mischungen isoliert.
Ausbeuten: Racemische Mischung 1: 12 mg; LC/MS (Methode J): m/z = 408 (M+1); Rt = 0,668 min
Racemische Mischung 2: 62 mg; LC/MS (Methode J): m/z = 408 (M+1); Rt = 0,665 min
Racemische Mischung 3: 18 mg; LC/MS (Methode J): m/z = 408 (M+1); Rt = 0,669 min
Racemische Mischung 4: 32 mg; LC/MS (Methode J): m/z = 408 (M+1); Rt = 0,672 min

### Beispiel 13

### 6-{(E)-2-[5-(3-Fluor-phenyl)-pyridin-2-yl]-vinyl}-8-methyl-hexahydro-pyrrolo[3',4':3,4]pyrrolo[2,1-c][1,4]oxazin-7,9-dion (3 racemische Mischungen)

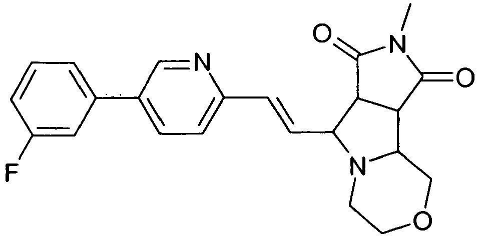

Die Titelverbindung wird in analoger Weise ausgehend von 100 mg (E)-3-[5-(3-Fluorphenyl)-pyridin-2-yl]-propenal, 49 mg N-Methylmaleinimid und 58 mg Morpholin-3-carbonsäure erhalten. Das Rohprodukt wird durch Säulenchromatografie (Kieselgel, MeOH:DCM = 2:98) gereinigt. Auf diese Weise werden 3 reine Faktionen als racemische Mischungen isoliert.
Ausbeuten: Racemische Mischung 1: 40 mg; LC/MS (Methode J): m/z = 408 (M+1); Rt = 0,738 min
Racemische Mischung 2: 39 mg; LC/MS (Methode J): m/z = 408 (M+1); Rt = 0,735 min
Racemische Mischung 3: 20 mg; LC/MS (Methode J): m/z = 408 (M+1); Rt = 0,697 min

### Beispiel 14

### 2-Ethyl-4-{(E)-2-[5-(3-fluor-phenyl)-pyridin-2-yl]-vinyl}-octahydro-pyrrolo[3,4-a]indolizin-1,3-dion (3 racemische Mischungen)

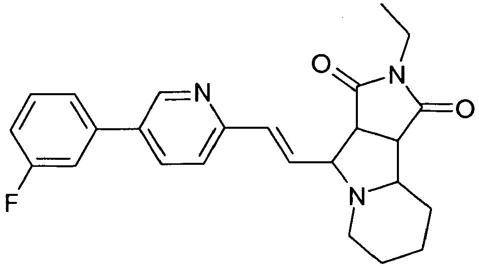

Die Titelverbindung wird in analoger Weise ausgehend von 100 mg (E)-3-[5-(3-Fluorphenyl)-pyridin-2-yl]-propenal, 55 mg N-Ethylmaleinimid und 57 mg D,L-Pipecolinsäure erhalten. Das Rohprodukt wird durch Säulenchromatografie (Kieselgel, MeOH:DCM = 1:99) gereinigt. Auf diese Weise werden 3 reine Faktionen als racemische Mischungen isoliert.
Ausbeuten: Racemische Mischung 1: 58 mg; LC/MS (Methode J): m/z = 420 (M+1); Rt = 0,747 min
Racemische Mischung 2: 16 mg; LC/MS (Methode J): m/z = 420 (M+1); Rt = 0,758 min
Racemische Mischung 3: 56 mg; LC/MS (Methode J): m/z = 420 (M+1); Rt = 0,745 min

### Beispiel 15

### 4-{(E)-2-[5-(3-Fluor-phenyl)-pyridin-2-yl]-vinyl}-2-methyl-hexahydro-pyrrolo[3,4-a]pyrrolizin-1,3-dion (3 racemische Mischungen)

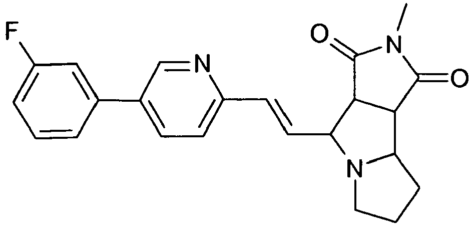

Die Titelverbindung wird in analoger Weise ausgehend von 100 mg (E)-3-[5-(3-Fluorphenyl)-pyridin-2-yl]-propenal, 49 mg N-Methylmaleinimid und 51 mg D,L-Prolin erhalten. Das Rohprodukt wird durch Säulenchromatografie (Kieselgel, MeOH:DCM = 1:99) gereinigt. Auf diese Weise werden 3 reine Faktionen als racemische Mischungen isoliert.
Ausbeuten: Racemische Mischung 1: 8 mg; LC/MS (Methode J): m/z = 392 (M+1); Rt = 0,955 min
Racemische Mischung 2: 57 mg; LC/MS (Methode J): m/z = 392 (M+1); Rt = 0,959 min
Racemische Mischung 3: 100 mg; LC/MS (Methode J): m/z = 392 (M+1); Rt = 0,954 min

### Beispiel 16

### 5-{(E)-2-[5-(3-Fluor-phenyl)-pyridin-2-yl]-vinyl}-7-methyl-tetrahydro-pyrrolo[3',4':3,4]pyrrolo[1,2-c]thiazol-6,8-dion (3 racemische Mischungen)

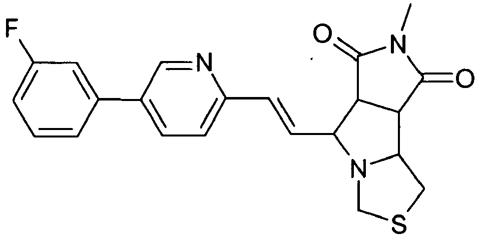

Die Titelverbindung wird in analoger Weise ausgehend von 100 mg (E)-3-[5-(3-Fluorphenyl)-pyridin-2-yl]-propenal, 49 mg N-Methylmaleinimid und 59 mg D-Thiazolidin-4-carbonsäure erhalten. Das Rohprodukt wird durch Säulenchromatografie (Kieselgel, MeOH:DCM = 3:97) gereinigt. Auf diese Weise werden 3 reine Faktionen als racemische Mischungen isoliert.
Ausbeuten: Racemische Mischung 1: 25 mg; LC/MS (Methode J): m/z = 410 (M+1); Rt = 0,814 min
Racemische Mischung 2: 37 mg; LC/MS (Methode J): m/z = 410 (M+1); Rt = 0,824 min
Racemische Mischung 3: 24 mg; LC/MS (Methode J): m/z = 410 (M+1); Rt = 0,809 min

### Beispiel 17

### 2-Methyl-4-{(E)-2-[5-(3-trifluormethyl-phenyl)-pyridin-2-yl]-vinyl}-octahydro-pyrrolo[3,4-a]indolizin-1,3-dion (racemische Mischung)

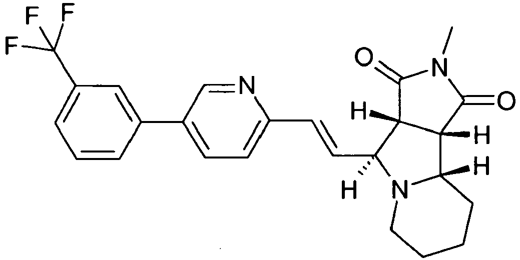

Die Mischung bestehend aus 42 mg 4-[(E)-2-(5-Brom-pyridin-2-yl)-vinyl]-2-methyl-octahydro-pyrrolo[3,4-a]indolizin-1,3-dion (aus Beispiel 6, racemische Mischung 1), 2,5 mg Tetrakis(triphenylphosphin)palladium(0), 25 mg 3-Trifluormethylphenylboronsäure, 37 mg Kaliumcarbonat, 1,5 ml Toluol, 0,3 ml Ethanol und 0,75 ml Wasser wird 5 h bei 100 °C gerührt. Nach dem Erkalten wird die Mischung in 20 ml Wasser aufgenommen und das Produkt mit 20 ml Essigester ausgeschüttelt. Nach dem Trocknen und Einengen der organischen Phase erhält man ein Öl. Das Rohprodukt wird durch Säulenchromatografie (Kieselgel, MeOH:DCM = 1:99) gereinigt.
Ausbeute: 36 mg; LC/MS (Methode J): m/z = 456 (M+1); Rt = 0,80 min

### Beispiel 18

### 2-Methyl-4-{(E)-2-[5-(3-trifluormethyl-phenyl)-pyridin-2-yl]-vinyl}-octahydro-pyrrolo[3,4-a]indolizin-1,3-dion (racemische Mischung)

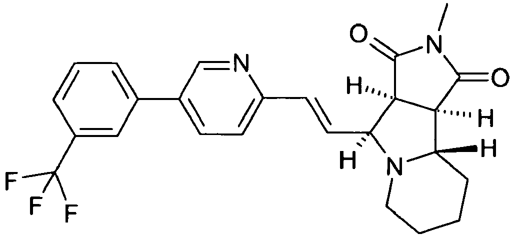

Die Titelverbindung wird in analoger Weise zur Verbindung des Beispiels 17 aus 45 mg 4-[(E)-2-(5-Brom-pyridin-2-yl)-vinyl]-2-methyl-octahydro-pyrrolo[3,4-a]indolizin-1,3-dion (aus Beispiel 6, racemische Mischung 3) und 26 mg 3-Trifluormethylphenylboronsäure hergestellt.
Ausbeute: 33 mg; LC/MS (Methode J): m/z = 456 (M+1); Rt = 0,799 min

### Beispiel 19

### 4-{(E)-2-[5-(2-Methoxy-phenyl)-pyridin-2-yl]-vinyl}-2-methyl-octahydro-pyrrolo[3,4-a]indolizin-1,3-dion (racemische Mischung)

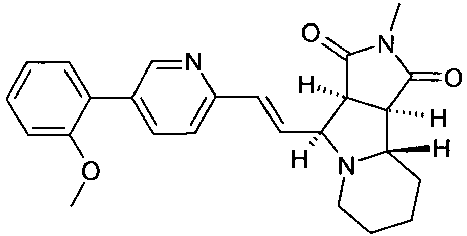

Die Titelverbindung wird in analoger Weise zur Verbindung des Beispiels 17 aus 42 mg 4-[(E)-2-(5-Brom-pyridin-2-yl)-vinyl]-2-methyl-octahydro-pyrrolo[3,4-a]indolizin-1,3-dion (aus Beispiel 6, racemische Mischung 3) und 18 mg 2-Methoxyphenylboronsäure hergestellt.
Ausbeute: 15 mg; LC/MS (Methode J): m/z = 418 (M+1); Rt = 0,674 min

### Beispiel 20

### 4-{(E)-2-[5-(2-Chlor-phenyl)-pyridin-2-yl]-vinyl}-2-methyl-octahydro-pyrrolo[3,4-a]indolizin-1,3-dion (racemische Mischung)

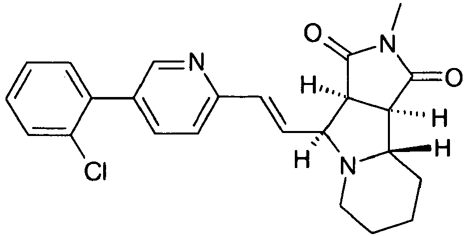

Die Titelverbindung wird in analoger Weise zur Verbindung des Beispiels 17 aus 42 mg 4-[(E)-2-(5-Brom-pyridin-2-yl)-vinyl]-2-methyl-octahydro-pyrrolo[3,4-a]indolizin-1,3-dion (aus Beispiel 6, racemische Mischung 3) und 18 mg 2-Chlorphenylboronsäure hergestellt.
Ausbeute: 7 mg; LC/MS (Methode D): m/z = 422 (M+1); Rt = 1,044 min

### Beispiel 21

### 2-Methyl-4-[(E)-2-(5-thiophen-3-yl-pyridin-2-yl)-vinyl]-octahydro-pyrrolo[3,4-a]indolizin-1,3-dion (racemische Mischung)

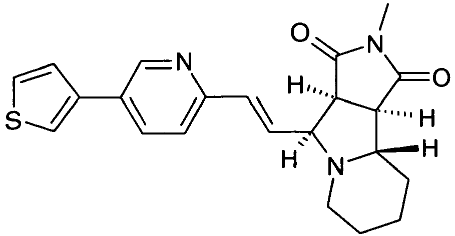

Die Titelverbindung wird in analoger Weise zur Verbindung des Beispiels 17 aus 42 mg 4-[(E)-2-(5-Brom-pyridin-2-yl)-vinyl]-2-methyl-octahydro-pyrrolo[3,4-a]indolizin-1,3-dion (aus Beispiel 6, racemische Mischung 3) und 15 mg 3-Thiophen-boronsäure hergestellt.
Ausbeute: 15 mg; LC/MS (Methode J): m/z = 394 (M+1); Rt = 0,617 min

### Beispiel 22

### 4-{(E)-2-[5-(3-Fluor-phenyl)-pyridin-2-yl]-vinyl}-2-methyl-3a,4,6,9,9a,9b-hexahydro-pyrrolo[3,4-a]indolizin-1,3-dion (3 racemische Mischungen)

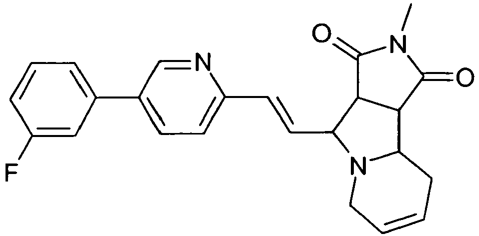

Die Titelverbindung wird in analoger Weise ausgehend von 100 mg (E)-3-[5-(3-Fluorphenyl)-pyridin-2-yl]-propenal, 49 mg N-Methylmaleinimid und 58 mg 1,2,3,6-Tetrahydro-pyridin-2-carbonsäure-hydrochlorid erhalten. Das Rohprodukt wird durch Säulenchromatografie (Kieselgel, MeOH:DCM = 2:98) gereinigt. Auf diese Weise werden 3 reine Faktionen als racemische Mischungen isoliert.
Ausbeuten: Racemische Mischung 1: 11 mg; LC/MS (Methode J): m/z = 404 (M+1); Rt = 0,687 min
Racemische Mischung 2: 10 mg; LC/MS (Methode J): m/z = 404 (M+1); Rt = 0,691 min
Racemische Mischung 3: 20 mg; LC/MS (Methode J): m/z = 404 (M+1); Rt = 0,6692 min

### Beispiel 23

### 2-Cyclopropyl-4-{(E)-2-[5-(3-fluor-phenyl)-pyridin-2-yl]-vinyl}-octahydro-pyrrolo[3,4-a]indolizin-1,3-dion (2 racemische Mischungen)

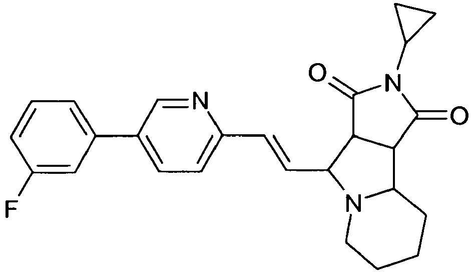

Die Titelverbindung wird in analoger Weise ausgehend von 80 mg (E)-3-[5-(3-Fluorphenyl)-pyridin-2-yl]-propenal, 30 mg N-Cyclopropylmaleinimid und 45 mg D,L-Pipecolinsäure erhalten. Das Rohprodukt wird durch Säulenchromatografie (Kieselgel, MeOH:DCM = 2:98) gereinigt. Auf diese Weise werden 2 reine Faktionen als racemische Mischungen isoliert.
Ausbeuten: Racemische Mischung 1: 50 mg; LC/MS (Methode J): m/z = 432 (M+1); Rt = 0,732 min
Racemische Mischung 2: 10 mg; LC/MS (Methode J): m/z = 432 (M+1); Rt = 0,736 min

### Beispiel 24

### 8-{(E)-2-[5-(3-Fluor-phenyl)-pyridin-2-yl]-vinyl}-2-methyl-octahydro-2,5,7a-triazacyclopenta[a]inden-1,3-dion-Trifluoressigsäuresalz

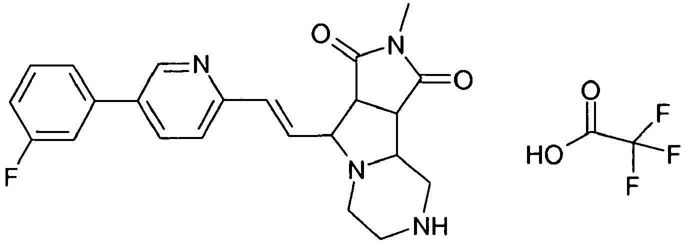

100 mg (8-{(E)-2-[5-(3-Fluor-phenyl)-pyridin-2-yl]-vinyl}-2-methyl-1,3-dioxo-decahydro-2,5,7a-triazacyclopenta[a]inden-5-carbonsäure-tert-butylester (aus Beispiel 10) werden in 9 mL Dichlormethan gelöst und mit 3 mL Trifluoressigsäure versetzt. Die Mischung wird 2 h bei RT gerührt, dann wird das Lösungsmittel unter verminderten Druck entfernt und das Rohprodukt durch Säulenchromatographie (Kieselgel, MeOH:DCM) gereinigt.
Ausbeute: 120 mg; LC/MS (Methode J): m/z = 407 (M+1); Rt = 0,595 min

### Pharmakologische Beispiele

### PAR1-Bestimmungsmethode: Hemmung der PAR1-mediierten Thrombozytenaggregation

Die pharmakologische Testung der Substanzen erfolgte in der durch TRAP (Thrombinrezeptor-aktivierendes Peptid) induzierten Thrombozytenaggregation im 96-well Format. Dazu wurde von gesunden Freiwilligen Blut in 20 ml Spritzen abgenommen, in denen 2 ml 3,13 %-ige Natriumcitratlösung vorgelegt war. Nach einer 20-minütigen Zentrifugation bei 150 x g wurde das Plättchenreiche Plasma (PRP) abgetrennt und mit 1 µl PGE1-Lösung (500 µg/ml in Ethanol) / ml PRP versetzt. Nach 5 Minuten Inkubation bei RT wurde 15 Minuten bei 120 x g zentrifugiert um die Leukozyten zu entfernen. Das Leukozytenfreie PRP wurde in 5 ml Portionen in 15 ml PP Röhrchen überführt und 15 Minuten bei 360xg abzentrifugiert, um die Plättchen zu pelletieren. Anschließend wurde das Plasma dekantiert und das Plättchensediment aus 5 ml PRP in 1 ml Tyrode (120 mM NaCl, 2,6 mM KCl, 12 mM NaHCO₃, 0,39 mM NaH₂PO₄ x H₂O, 10 mM HEPES, 0,35% BSA (Bovine Serum Albumin), 5,5 mM Glukose, pH 7,4) resuspendiert und mit Tyrode auf eine Plättchenzahl von 3x105 / Mikroliter (µL) eingestellt. 13 ml dieser Zellsuspension wurde dann mit 866 µL 10 mM CaCl₂-Lösung versetzt und 120 µL davon pro well einer 96-well-Platte pipettiert, in dem 15 µL der zu testenden Substanz vorgelegt waren. Nach 30 Minuten Inkubation bei RT im Dunklen wurden 15 µL einer TRAP-Lösung (70-100 µM) als Agonist zugegeben und in einem SpectraMax 340 bei 650 nm über 20 Minuten bei 37° C unter Schütteln eine Kinetik aufgezeichnet. Die Flächen unter den Kurven von Negativkontrolle (Tyrode/DMSO) und Positivkontrolle (15 µl Agonist DMSO) wurden berechnet und die Differenz als 100 % Wert festgelegt. Die zu testenden Substanzen wurden in Doppelbestimmung als Verdünnungsreihen pipettiert, ebenfalls die AUC jeder Substanzkonzentration bestimmt und die % Hemmung der AUC gegen die Kontrolle errechnet. Anhand der % Hemmung wurde mit Hilfe nichtlinearer Regressionsanalyse gemäß der 4 Parameter-Gleichung die IC₅₀-Werte berechnet. Tabelle 1 zeigt Ergebnisse (IC₅₀-Werte in mikromol/L).

**Tabelle 1**

| Verbindung aus Beispiel | Hemmung der Thrombozytenaggregation IC₅₀ [mikro M] | Verbindung aus Beispiel | Hemmung der Thrombozytenaggregation IC₅₀ [mikro M] |
|---|---|---|---|
| 11 (Fr. 2) | 15,8 | 18 | 3,23 |
| 13 (Fr. 3) | 7,1 | 19 | 4,09 |
| 14 (Fr. 3) | 3,68 | 20 | 0,26 |

| | | | |
|---|---|---|---|
| "Fr." bezeichnet die Nummer der im Beispiel beschriebenen racemischen Mischung (Fraktion) | | | |

## Patentansprüche

1. Verbindung der Formel I, 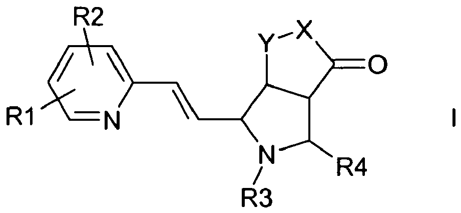 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₆)-Alkyl, -O-(C₁-C₆)-Alkyl, Aryl, Halogen oder Hetaryl stehen, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂, -NH₂, -OH, -O-CF₃, -S-CF₃ oder -CF₃ substituiert sind, oder
R1 und R2 zusammen mit den Ringatomen, an die sie jeweils gebunden sind, einen 5-gliedrigen bis 6-gliedrigen Ring bilden, wobei der Ring nur aus Kohlenstoffatomen besteht oder 1, 2 oder 3 dieser Atome durch N-, O- oder S-Atome ersetzt sind, wobei der Ring unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂, -NH₂, -OH, -O-CF₃, -S-CF₃ oder -CF₃ substituiert ist;
R3 und R4 zusammen mit den Ringatomen, an die sie jeweils gebunden sind, einen gesättigten oder ungesättigten 5-gliedrigen bis 7-gliedrigen Ring bilden, wobei der Ring nur aus Kohlenstoffatomen besteht oder 1 oder 2 dieser Atome durch O, S, SO, SO₂ oder N-R5 ersetzt sind, wobei der Ring unsubstituiert oder einfach, zweifach oder dreifach unabhängig voneinander durch F, Cl, -OH, -NH-R7, -(C₁-C₄)-Alkyl, -O-(C₁-C₄)-Alkyl, C(O)-O-(C₁-C₄)-Alkyl, Aryl oder Hetaryl substituiert ist;
X für N-R6 oder O steht;
Y für C(O), CH₂, CH-CH₃ oder C(CH₃)₂ steht;
R5 für Wasserstoff, -C(O)-(C₁-C₄)-Alkyl, -C(O)-O-(C₁-C₄)-Alkyl, -(C₁-C₆)-Alkyl, -(C₂-C₄)-Alkyl-O-(C₁-C₄)-Alkyl, -(C₂-C₄)-Alkyl-OH, Aryl oder Hetaryl steht;
R7 für Wasserstoff, -C(O)-(C₁-C₄)-Alkyl, -C(O)-O-(C₁-C₄)-Alkyl, -(C₁-C₆)-Alkyl, -(C₂-C₄)-Alkyl-O-(C₁-C₄)-Alkyl, -(C₂-C₄)-Alkyl-OH, Aryl oder Hetaryl steht;
R6 für Wasserstoff, -(C₁-C₆)-Alkyl, -(C₃-C₇)-Cycloalkyl, -(C₁-C₄)-Alkylen-Aryl oder C(O)-O-(C₁-C₄)-Alkyl steht;
wobei unter dem Begriff "Hetaryl" Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten.

2. Verbindung der Formel I gemäß Anspruch 1, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₆)-Alkyl, -O-(C₁-C₆)-Alkyl, Aryl, Halogen oder Hetaryl stehen, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl, -NH-(C₁-C₄)-Alkyl, -N((C₁-C₄)-Alkyl)₂ oder -CF₃ substituiert sind, oder
R1 und R2 zusammen mit den Ringatomen, an die sie jeweils gebunden sind, einen Ring bilden, wobei das bicyclische Ringsystem, das aus diesem Ring und dem R1 und R2 tragenden Pyridinring besteht, ausgewählt ist aus der Gruppe Chinolin und Isochinolin;
R3 und R4 zusammen mit den Ringatomen, an die sie jeweils gebunden sind, einen gesättigten oder ungesättigten 5-gliedrigen bis 7-gliedrigen Ring bilden, wobei der Ring nur aus Kohlenstoffatomen besteht oder 1 oder 2 dieser Atome durch O, S, SO, SO₂ oder N-R5 ersetzt sind, ausgewählt aus der Gruppe Azepin, [1,2]Diazepin, [1,3]Diazepin, [1,4]Diazepin, Dihydroimidazolon, Imidazol, Imidazolin, Imidazolidin, Imidazolidinon, Isothiazol, Isothiazolidin, Isothiazolin, Isoxazol, Isoxazolin, Isoxazolidin, Morpholin, Oxathiazindioxid, [1,2]Oxazin, [1,3]Oxazin, [1,4]Oxazin, Oxazolon, Oxazol, Oxazolidin, Piperazin, Piperidin, Pyrazin, Pyrazol, Pyrazolin, Pyrazolidin, Pyridazin, Pyridin, Pyridinon, Pyridopyrazine, Pyridopyridine, Pyrimidin, Pyrrol, Pyrrolidin, Pyrrolidinon, Pyrrolin, 1,2,3,6-Tetrahydro-pyridin, [1,2]Thiazin, [1,3]Thiazin, [1,4]Thiazin, [1,3]Thiazol, Thiazol oder Thiazolidin, wobei der Ring unsubstituiert oder einfach, zweifach oder dreifach unabhängig voneinander durch F, Cl, -OH, -(C₁-C₄)-Alkyl, -O-(C₁-C₄)-Alkyl, C(O)-O-(C₁-C₄)-Alkyl, Aryl oder Hetaryl substituiert ist;
X für N-R6 oder O steht;
Y für C(O), CH₂ oder CH-CH₃ steht;
R5 für Wasserstoff, C(O)-(C₁-C₄)-Alkyl, C(O)-O-(C₁-C₄)-Alkyl, -(C₁-C₆)-Alkyl, Aryl oder Hetaryl steht;
R6 für Wasserstoff, -(C₁-C₆)-Alkyl, -(C₃-C₇)-Cycloalkyl oder -(C₁-C₄)-Alkylen-Aryl steht;
wobei unter dem Begriff "Hetaryl" Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten.

3. Verbindung der Formel I gemäß den Ansprüchen 1 bis 2, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₄)-Alkyl, -O-(C₁-C₄)-Alkyl, Br, F, Cl, Aryl oder Hetaryl stehen, wobei Hetaryl ausgewählt wird aus der Gruppe Acridinyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H,6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxothiolanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrolyl, Thienopyridinyl, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl und Xanthenyl, wobei Alkyl, Aryl und Hetaryl jeweils unsubstituiert oder einfach, zweifach oder dreifach unabhängig voneinander durch F, Cl, CN, -(C₁-C₄)-Alkyl, -O-(C₁-C₄)-Alkyl, -N((C₁-C₄)-Alkyl)₂ oder -CF₃ substituiert sind;
R3 und R4 zusammen mit den Ringatomen, an die sie jeweils gebunden sind, einen gesättigten oder ungesättigten 5-gliedrigen bis 7-gliedrigen Ring bilden, ausgewählt aus der Gruppe Azepin, [1,2]Diazepin, [1,3]Diazepin, [1,4]Diazepin, Dihydroimidazolon, Imidazol, Imidazolin, Imidazolidin, Imidazolidinon, Isothiazol, Isothiazolidin, Isothiazolin, Isoxazol, Isoxazolin, Isoxazolidin, Morpholin, Oxathiazindioxid, [1,2]Oxazin, [1,3]Oxazin, [1,4]Oxazin, Oxazolon, Oxazol, Oxazolidin, Piperazin, Piperidin, Pyrazin, Pyrazol, Pyrazolin, Pyrazolidin, Pyridazin, Pyridin, Pyridinon, Pyrimidin, Pyrrol, Pyrrolidin, Pyrrolidinon, Pyrrolin, 1,2,3,6-Tetrahydro-pyridin, [1,2]Thiazin, [1,3]Thiazin, [1,4]Thiazin, [1,3]Thiazol, Thiazol und Thiazolidin, wobei der Ring unsubstituiert oder einfach, zweifach oder dreifach unabhängig voneinander durch F, Cl, -OH, -(C₁-C₄)-Alkyl, C(O)-O-(C₁-C₄)-Alkyl, Aryl oder Hetaryl substituiert ist;
X für N-R6 oder O steht;
Y für C(O), CH₂ oder CH-CH₃ steht;
R6 für Wasserstoff, -(C₁-C₆)-Alkyl, -(C₃-C₆)-Cycloalkyl oder -(C₁-C₄)-Alkylen-Aryl steht.

4. Verbindung der Formel I gemäß den Ansprüchen 1 bis 3, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₄)-Alkyl, Br, Thienyl oder Phenyl stehen, wobei Thienyl und Phenyl jeweils unsubstituiert oder einfach oder zweifach unabhängig voneinander durch F, Cl, -O-Methyl oder -CF₃ substituiert sind;
R3 und R4 zusammen mit den Ringatomen, an die sie jeweils gebunden sind, einen gesättigten oder ungesättigten 5-gliedrigen bis 6-gliedrigen Ring bilden, ausgewählt aus der Gruppe Azepin, [1,2]Diazepin, [1,3]Diazepin, [1,4]Diazepin, Dihydroimidazolon, Imidazol, Imidazolin, Imidazolidin, Imidazolidinon, Isothiazol, Isothiazolidin, Isothiazolin, Isoxazol, Isoxazolin, Isoxazolidin, Morpholin, Oxathiazindioxid, [1,2]Oxazin, [1,3]Oxazin, [1,4]Oxazin, Oxazolon, Oxazol, Oxazolidin, Piperazin, Piperidin, Pyrazin, Pyrazol, Pyrazolin, Pyrazolidin, Pyridazin, Pyridin, Pyridinon, Pyrimidin, Pyrrol, Pyrrolidin, Pyrrolidinon, Pyrrolin, 1,2,3,6-Tetrahydro-pyridin, [1,2]Thiazin, [1,3]Thiazin, [1,4]Thiazin, [1,3]Thiazol, Thiazol und Thiazolidin, wobei der Ring unsubstituiert oder einfach, zweifach oder dreifach unabhängig voneinander durch F, -OH, C(O)-O-(C₁-C₄)-Alkyl, Phenyl oder Hetaryl substituiert ist;
X für N-R6 oder O steht;
Y für C(O), CH₂ oder CH-CH₃ steht;
R6 für Wasserstoff, -(C₁-C₆)-Alkyl, -(C₃-C₆)-Cycloalkyl oder -(C₁-C₄)-Alkylen-Phenyl steht.

5. Verbindung der Formel I gemäß den Ansprüchen 1 bis 4, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₄)-Alkyl, Br, Thienyl oder Phenyl stehen, wobei Thienyl und
Phenyl jeweils unsubstituiert oder einfach oder zweifach unabhängig voneinander durch F, Cl, -O-Methyl oder -CF₃ substituiert sind,
R3 und R4 zusammen mit den Ringatomen, an die sie jeweils gebunden sind, einen gesättigten oder ungesättigten 5-gliedrigen bis 6-gliedrigen Ring bilden, ausgewählt aus der Gruppe Morpholin, Piperazin, Piperidin, Pyrrolidin, 1,2,3,6-Tetrahydro-pyridin oder [1,3]-Thiazol, wobei der Ring unsubstituiert oder einfach, zweifach oder dreifach unabhängig voneinander durch F, -OH, Phenyl oder C(O)-O-(C₁-C₄)-Alkyl substituiert ist;
X für N-R6 steht;
Y für C(O), CH₂ oder CH-CH₃ steht;
R6 für Wasserstoff, -(C₁-C₆)-Alkyl, Cyclopropyl oder -(C₁-C₄)-Alkylen-Phenyl steht.

6. Verbindung der Formel I gemäß den Ansprüchen 1 bis 5, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei die Verbindung der Formel I ausgewählt ist aus der Gruppe
4-{((E)-2-[5-(3-Fluor-phenyl)-pyridin-2-yl]-vinyl}-2-methyl-octahydro-pyrrolo[3,4-a]indolizin-1,3-dion,
4-[((E)-2-(5-Brom-pyridin-2-yl)-vinyl]-2-methyl-octahydro-pyrrolo[3,4-a]indolizin-1,3-dion,
7-Hydroxy-2-methyl-4-[(E)-2-(6-methyl-pyridin-2-yl)-vinyl]-hexahydro-pyrrolo[3,4-a]-pyrrolizin-1,3-dion,
2-Methyl-4-[(E)-2-(6-methyl-pyridin-2-yl)-vinyl]-octahydro-pyrrolo[3,4-a]-indolizin-1,3-dion,
2-Methyl-4-[(E)-2-(6-methyl-pyridin-2-yl)-vinyl]-hexahydro-pyrrolo[3,4-a]-pyrrolizin-1,3-dion,
(8-{((E)-2-[5-(3-Fluor-phenyl)-pyridin-2-yl]-vinyl}-2-methyl-1,3-dioxo-decahydro-2,5,7a-triazacyclopenta[a]inden-5-carbonsäure-tert-butylester,
(S)-4-{(E)-2-[5-(3-Fluor-phenyl)-pyridin-2-yl]-vinyl}-7-hydroxy-2-methyl-hexahydro-pyrrolo[3,4-a]pyrrolizin-1,3-dion,
(R)-4-{(E)-2-[5-(3-Fluor-phenyl)-pyridin-2-yl]-vinyl}-7-hydroxy-2-methyl-hexahydro-pyrrolo[3,4-a]pyrrolizin-1,3-dion,
6-{((E)-2-[5-(3-Fluor-phenyl)-pyridin-2-yl]-vinyl}-8-methyl-hexahydro-pyrrolo[3',4':3,4]pyrrolo[2,1-c][1,4]oxazin-7,9-dion,
2-Ethyl-4-{(E)-2-[5-(3-fluor-phenyl)-pyridin-2-yl]-vinyl}-octahydro-pyrrolo[3,4-a]indolizin-1,3-dion,
4-{((E)-2-[5-(3-Fluor-phenyl)-pyridin-2-yl]-vinyl}-2-methyl-hexahydro-pyrrolo[3,4-a]pyrrolizin-1,3-dion,
5-{((E)-2-[5-(3-Fluor-phenyl)-pyridin-2-yl]-vinyl}-7-methyl-tetrahydro-pyrrolo[3',4':3,4]pyrrolo[1,2-c]thiazol-6,8-dion,
2-Methyl-4-{(E)-2-[5-(3-trifluormethyl-phenyl)-pyridin-2-yl]-vinyl}-octahydro-pyrrolo[3,4-a]indolizin-1,3-dion,
4-{((E)-2-[5-(2-Methoxy-phenyl)-pyridin-2-yl]-vinyl}-2-methyl-octahydro-pyrrolo[3,4-a]indolizin-1,3-dion,
4-{((E)-2-[5-(2-Chlor-phenyl)-pyridin-2-yl]-vinyl}-2-methyl-octahydro-pyrrolo[3,4-a]indolizin-1,3-dion,
2-Methyl-4-[(E)-2-(5-thiophen-3-yl-pyridin-2-yl)-vinyl]-octahydro-pyrrolo[3,4-a]indolizin-1,3-dion,
4-{((E)-2-[5-(3-Fluor-phenyl)-pyridin-2-yl]-vinyl}-2-methyl-3a,4,6,9,9a,9b-hexahydro-pyrrolo[3,4-a]indolizin-1,3-dion,
2-Cyclopropyl-4-{(E)-2-[5-(3-fluor-phenyl)-pyridin-2-yl]-vinyl}-octahydro-pyrrolo[3,4-a]indolizin-1,3-dion, und
8-{((E)-2-[5-(3-Fluor-phenyl)-pyridin-2-yl]-vinyl}-2-methyl-octahydro-2,5,7a-triazacyclopenta[a]inden-1,3-dion.

7. Arzneimittel, **gekennzeichnet durch** einen wirksamen Gehalt an mindestens einer Verbindung der Formel I oder eines physiologisch verträglichen Salzes davon gemäß einem oder mehreren der Ansprüche 1 bis 6 zusammen mit einem pharmazeutisch geeigneten und physiologisch verträglichen Trägerstoff, Zusatzstoff und/oder anderen Wirkstoffen und Hilfsstoffen.

8. Verbindung der Formel I oder ein physiologisch verträgliches Salz davon gemäß einem oder mehreren der Ansprüche 1 bis 6 zur Verwendung als Pharmazeutikum.

9. Verbindung der Formel I oder ein physiologisch verträgliches Salz davon gemäß einem oder mehreren der Ansprüche 1 bis 6 zur Verwendung für die Prophylaxe, Sekundärprevention und Therapie all solcher Erkrankungen, die mit Thrombosen, Embolien, Hyperkoagulabilität, fibrotischen Veränderungen oder entzündlichen Erkrankungen einhergehen.

10. Verbindung der Formel I oder ein physiologisch verträgliches Salz davon gemäß einem oder mehreren der Ansprüche 1 bis 6 zur Verwendung gemäß Anspruch 9, **dadurch gekennzeichnet, dass** es sich um Myokardinfarkt, Angina pectoris und andere Formen des akuten Koronarsyndroms, den akuten Schlaganfall oder dessen Sekundärprevention, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen, die Reduktion der Thrombosegefahr nach chirurgischen Eingriffen wie bei Knieoperationen und Hüftgelenksoperationen, Eingriffe, die zu einem Kontakt des Blutes mit Fremdoberflächen führen wie bei Dialysepatienten und Patienten mit Verweilkathetern, die disseminierte intravaskuläre Koagulation, Sepsis und andere intravaskuläre Ereignissen, die mit einer Entzündung einhergehen, Atherosklerose, Diabetes und das metabolische Syndrom und deren Folgen, Tumorwachstum und Tumormetastasierung, entzündliche und degenerative Gelenkserkrankungen wie die rheumatoide Arthritis und die Arthrose, Störungen des hämostatischen Systems wie Fibrinablagerungen, fibrotische Veränderungen der Lunge wie die chronische obstruktive Lungenerkrankung, das adult respiratory distress syndrom, Fibrinablagerungen des Auges nach Augenoperationen oder Verhinderung und/oder Behandlung von Narbenbildung.

11. Verfahren zur Herstellung einer Verbindung der Formel I oder eines physiologisch verträglichen Salzes davon gemäß einem oder mehreren der Ansprüche 1 bis 6, **dadurch gekennzeichnet ist, dass** man
a) eine Verbindung der Formel II, 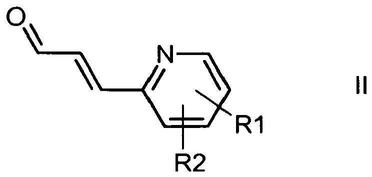 wobei die Reste R1 und R2 wie in Formel I definiert sind, mit einer Verbindung der Formel III und einer Verbindung der Formel IV, 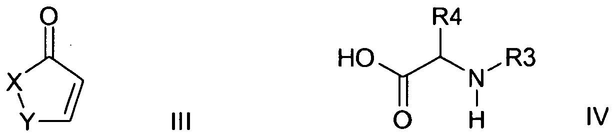 wobei die Reste X, Y, R3 und R4 wie in Formel I definiert sind, in Gegenwart eines Lösungsmittels bei 20°C bis 120°C zu einer Verbindung der Formel I umsetzt; oder
b) eine Verbindung der Formel V, 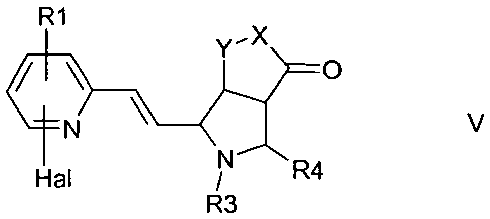 wobei die Reste X, Y, R1, R3 und R4 wie in Formel I definiert sind und Hal die Bedeutung Chlor, Brom, Iod oder Triflat hat, mit einer Verbindung der Formel R2-B(OH)₂ in Gegenwart einer Base und eines geeigneten Metallkatalysators in einem geeigneten Lösemittel oder Lösemittelgemisch zu einer Verbindung der Formel I umsetzt; oder
c) eine Verbindung der Formel I, worin X die Bedeutung NH hat, mit einem geeigneten Alkylierungsmittel in Gegenwart einer Base und in einem geeigneten inerten Lösemittel, bei Raumtemperatur oder bei erhöhter Temperatur umsetzt zu der Verbindung der Formel I, worin X die Bedeutung N-R6 hat und R6 -(C₁-C₆)-Alkyl, -(C₃-C₇)-Cycloalkyl oder -(C₁-C₄)-Alkylen-Aryl bedeutet; oder
d) die nach den Verfahren a) bis c) hergestellte Verbindung der Formel I, oder eine geeignete Vorstufe der Formel I, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt; oder
e) die nach den Verfahren a) bis d) hergestellte Verbindung der Formel I entweder in freier Form isoliert, oder aus physiologisch unverträglichen Salzen freisetzt, oder im Falle des Vorliegens von sauren oder basischen Gruppen in ein physiologisch verträgliches Salz umwandelt.

## Claims

1. A compound of the formula I 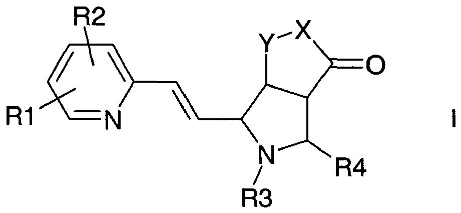 and/or all stereoisomeric or tautomeric forms of the compound of the formula I and/or mixtures of these forms in any ratio, and/or a physiologically acceptable salt of the compound of the formula I, where
R1 and R2 are identical or different and are independently of one another hydrogen, -(C₁-C₆)-alkyl, -O-(C₁-C₆)-alkyl, aryl, halogen or hetaryl, where alkyl, aryl and hetaryl are in each case unsubstituted or mono-, di- or trisubstituted independently of one another by F, Cl, Br, CN, -(C₁-C₄)-alkyl, -O-(C₁-C₄)-alkyl, -NH-(C₁-C₄)-alkyl, -N((C₁-C₄)-alkyl)₂, -NH₂, -OH, -O-CF₃, -S-CF₃ or -CF₃, or
R1 and R2 together with the ring atoms to which they are respectively attached form a 5-membered to 6-membered ring, where the ring consists only of carbon atoms, or 1, 2 or 3 of these atoms are replaced by nitrogen, oxygen or sulfur atoms, where the ring is unsubstituted or mono-, di- or trisubstituted independently of one another by F, Cl, Br, CN, -(C₁-C₄)-alkyl, -O-(C₁-C₄)-alkyl, -NH-(C₁-C₄)-alkyl, -N((C₁-C₄)-alkyl)₂, -NH₂, -OH, -O-CF₃, -S-CF₃ or -CF₃;
R3 and R4 together with the ring atoms to which they are respectively attached form a saturated or unsaturated 5-membered to 7-membered ring, where the ring consists only of carbon atoms, or 1 or 2 of these atoms are replaced by O, S, SO, SO₂ or N-R5, where the ring is unsubstituted or mono-, di- or trisubstituted independently of one another by F, Cl, -OH, -NH-R7, -(C₁-C₄)-alkyl, -O-(C₁-C₄)-alkyl, C(O)-O-(C₁-C₄)-alkyl, aryl or hetaryl;
X is N-R6 or O;
Y is C(O), CH₂, CH-CH₃ or C(CH₃)₂;
R5 is hydrogen, -C(O)-(C₁-C₄)-alkyl, -C(O)-O-(C₁-C₄)-alkyl, -(C₁-C₆)-alkyl, -(C₂-C₄)-alkyl-O-(C₁-C₄)-alkyl, -(C₂-C₄)-alkyl-OH, aryl or hetaryl;
R7 is hydrogen, -C(O)-(C₁-C₄)-alkyl, -C(O)-O-(C₁-C₄)-alkyl, -(C₁-C₆)-alkyl, -(C₂-C₄)-alkyl-O-(C₁-C₄)-alkyl, -(C₂-C₄)-alkyl-OH, aryl or hetaryl;
R6 is hydrogen, -(C₁-C₆)-alkyl, -(C₃-C₇)-cycloalkyl, -(C₁-C₄)-alkylene-aryl or C(O)-O-(C₁-C₄)-alkyl;
where the term "hetaryl" means ring systems having 4 to 15 carbon atoms which are present in one, two or three ring systems connected together, and which comprise, depending on the ring size, one, two, three or four identical or different heteroatoms from the group consisting of oxygen, nitrogen and sulfur.

2. The compound of the formula I as claimed in claim 1, and/or all stereoisomeric or tautomeric forms of the compound of the formula I and/or mixtures of these forms in any ratio, and/or a physiologically acceptable salt of the compound of the formula I, where
R1 and R2 are identical or different and are independently of one another hydrogen, -(C₁-C₆)-alkyl, -O-(C₁-C₆)-alkyl, aryl, halogen or hetaryl, where alkyl, aryl and hetaryl are in each case unsubstituted or mono-, di- or trisubstituted independently of one another by F, Cl, Br, CN, -(C₁-C₄)-alkyl, -O-(C₁-C₄)-alkyl, -NH-(C₁-C₄)-alkyl, -N((C₁-C₄)-alkyl)₂ or -CF₃, or
R1 and R2 together with the ring atoms to which they are respectively attached form a ring, where the bicyclic ring system consisting of this ring and the pyridine ring carrying R1 and R2 is selected from the group consisting of quinoline and isoquinoline;
R3 and R4 together with the ring atoms to which they are respectively attached form a saturated or unsaturated 5-membered to 7-membered ring, where the ring consists only of carbon atoms or 1 or 2 of these atoms are replaced by O, S, SO, SO₂ or N-R5, selected from the group consisting of azepine, [1,2]diazepine, [1,3]diazepine, [1,4]diazepine, dihydroimidazolone, imidazole, imidazoline, imidazolidine, imidazolidinone, isothiazole, isothiazolidine, isothiazoline, isoxazole, isoxazoline, isoxazolidine, morpholine, oxathiazine dioxide, [1,2]oxazine, [1,3]oxazine, [1,4]oxazine, oxazolone, oxazole, oxazolidine, piperazine, piperidine, pyrazine, pyrazole, pyrazoline, pyrazolidine, pyridazine, pyridine, pyridinone, pyridopyrazines, pyridopyridines, pyrimidine, pyrrole, pyrrolidine, pyrrolidinone, pyrroline, 1,2,3,6-tetrahydropyridine, [1,2]thiazine, [1,3]thiazine, [1,4]thiazine, [1,3]thiazole, thiazole or thiazolidine, where the ring is unsubstituted or mono-, di- or trisubstituted independently of one another by F, Cl, -OH, -(C₁-C₄)-alkyl, -O-(C₁-C₄)-alkyl, C(O)-O-(C₁-C₄)-alkyl, aryl or hetaryl;
X is N-R6 or O;
Y is C(O), CH₂ or CH-CH₃;
R5 is hydrogen, C(O)-(C₁-C₄)-alkyl, C(O)-O-(C₁-C₄)-alkyl, -(C₁-C₆)-alkyl, aryl or hetaryl;
R6 is hydrogen, -(C₁-C₆)-alkyl, -(C₃-C₇)-cycloalkyl or -(C₁-C₄)-alkylene-aryl;
where the term "hetaryl" means ring systems having 4 to 15 carbon atoms which are present in one, two or three ring systems connected together, and which comprise, depending on the ring size, one, two, three or four identical or different heteroatoms from the group consisting of oxygen, nitrogen and sulfur.

3. The compound of the formula I as claimed in claims 1 and 2, and/or all stereoisomeric or tautomeric forms of the compound of the formula I and/or mixtures of these forms in any ratio, and/or a physiologically acceptable salt of the compound of the formula I, where
R1 and R2 are identical or different and are independently of one another hydrogen, -(C₁-C₄)-alkyl, -O-(C₁-C₄)-alkyl, Br, F, Cl, aryl or hetaryl, where hetaryl is selected from the group consisting of acridinyl, azepinyl, azetidinyl, aziridinyl, benzimidazolyl, benzofuranyl, benzothiofuranyl, benzothiophenyl, benzoxazolyl, benzthiazolyl, benztriazolyl, benzisoxazolyl, benzisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, deca-hydroquinolinyl, dibenzofuranyl, dibenzothiophenyl, dihydrofuran[2,3-b]-tetrahydrofuranyl, dihydrofuranyl, dioxolyl, dioxanyl, 2H,6H-1,5,2-dithiazinyl, furanyl, furazanyl, imidazolidinyl, imidazolinyl, imidazolyl, 1 H-indazolyl, indolinyl, indolizinyl, indolyl, 3H-indolyl, isobenzofuranyl, isoquinolinyl, isochromanyl, isoindazolyl, isoindolinyl, isoindolyl, isothiazolidinyl, 2-isothiazolinyl, isothiazolyl, isoxazolyl, isoxazolidinyl, 2-isoxazolinyl, morpholinyl, naphthyridinyl, octahydroisoquinolinyl, 1,2,3-oxadiazolyl, 1,2,4-oxadiazolyl, 1,2,5-oxadiazolyl, 1,3,4-oxadiazolyl, oxazolidinyl, oxazolyl, oxothiolanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyroazolidinyl, pyrazolinyl, pyrazolyl, pyridazinyl, pryidooxazolyl, pyridoimidazolyl, pyridothiazolyl, pyridothiophenyl, pyridyl,
pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolidinyl, thiazolinyl, thiazolyl, thienyl, thienoimidazolyl, thienooxazolyl, thienopyrrolyl, thienopyridinyl, thienothiazolyl, thienothiophenyl, thiomorpholinyl, triazinyl, 1,2,3-triazolyl, 1,2,4-triazolyl, 1,2,5-triazolyl, 1,3,4-triazolyl and xanthenyl, where alkyl, aryl and hetaryl are in each case unsubstituted or mono-, di- or trisubstituted independently of one another by F, Cl, CN, -(C₁-C₄)-alkyl, -O-(C₁-C₄)-alkyl, -N((C₁-C₄)-alkyl)₂ or -CF₃;
R3 and R4 together with the ring atoms to which they are respectively attached form a saturated or unsaturated 5-membered to 7-membered ring selected from the group consisting of azepine, [1,2]diazepine, [1,3]diazepine, [1,4]diazepine, dihydroimidazolone, imidazole, imidazoline, imidazolidine, imidazolidinone, isothiazole, isothiazolidine, isothiazoline, isoxazole, isoxazoline, isoxazolidine, morpholine, oxathiazine dioxide, [1,2]oxazine, [1,3]oxazine, [1,4]oxazine, oxazolone, oxazole, oxazolidine, piperazine, piperidine, pyrazine, pyrazole, pyrazoline, pyrazolidine, pyridazine, pyridine, pyridinone, pyrimidine, pyrrole, pyrrolidine, pyrrolidinone, pyrroline, 1,2,3,6-tetrahydropyridine, [1,2]thiazine, [1,3]thiazine, [1,4]thiazine, [1,3]thiazole, thiazole and thiazolidine, where the ring is unsubstituted or mono-, di- or trisubstituted independently of one another by F, Cl, -OH, -(C₁-C₄)-alkyl, C(O)-O-(C₁-C₄)-alkyl, aryl or hetaryl;
X is N-R6 or O;
Y is C(O), CH₂ or CH-CH₃;
R6 is hydrogen, -(C₁-C₆)-alkyl, -(C₃-C₆)-cycloalkyl or -(C₁-C₄)-alkylene-aryl.

4. The compound of the formula I as claimed in any of claims 1 to 3, and/or all stereoisomeric or tautomeric forms of the compound of the formula I and/or mixtures of these forms in any ratio, and/or a physiologically acceptable salt of the compound of the formula I, where
R1 and R2 are identical or different and are independently of one another hydrogen, -(C₁-C₄)-alkyl, Br, thienyl or phenyl, where thienyl and phenyl are in each case unsubstituted or mono- or disubstituted independently of one another by F, Cl, -O-methyl or -CF₃;
R3 and R4 together with the ring atoms to which they are respectively attached form a saturated or unsaturated 5-membered to 6-membered ring selected from the group consisting of azepine, [1,2]diazepine, [1,3]diazepine, [1,4]diazepine, dihydroimidazolone, imidazole, imidazoline, imidazolidine, imidazolidinone, isothiazole, isothiazolidine, isothiazoline, isoxazole, isoxazoline, isoxazolidine, morpholine, oxathiazine dioxide, [1,2]oxazine, [1,3]oxazine, [1,4]oxazine, oxazolone, oxazole, oxazolidine, piperazine, piperidine, pyrazine, pyrazole, pyrazoline, pyrazolidine, pyridazine, pyridine, pyridinone, pyrimidine, pyrrole, pyrrolidine, pyrrolidinone, pyrroline, 1,2,3,6-tetrahydropyridine, [1,2]thiazine, [1,3]thiazine, [1,4]thiazine, [1,3]thiazole, thiazole and thiazolidine, where the ring is unsubstituted or mono-, di- or trisubstituted independently of one another by F, -OH, C(O)-O-(C₁-C₄)-alkyl, phenyl or hetaryl;
X is N-R6 or O;
Y is C(O), CH₂ or CH-CH₃;
R6 is hydrogen, -(C₁-C₆)-alkyl, -(C₃-C₆)-cycloalkyl or -(C₁-C₄)-alkylene-phenyl.

5. The compound of the formula I as claimed in any of claims 1 to 4, and/or all stereoisomeric or tautomeric forms of the compound of the formula I and/or mixtures of these forms in any ratio, and/or a physiologically acceptable salt of the compound of the formula I, where
R1 and R2 are identical or different and are independently of one another hydrogen, -(C₁-C₄)-alkyl, Br, thienyl or phenyl, where thienyl and phenyl are in each case unsubstituted or mono- or disubstituted independently of one another by F, Cl, -O-methyl or -CF₃;
R3 and R4 together with the ring atoms to which they are respectively attached form a saturated or unsaturated 5-membered to 6-membered ring selected from the group consisting of morpholine, piperazine, piperidine, pyrrolidine, 1,2,3,6-tetrahydropyridine and [1,3]thiazole, where the ring is unsubstituted or mono-, di- or trisubstituted independently of one another by F, -OH, phenyl or C(O)-O-(C₁-C₄)-alkyl;
X is N-R6;
Y is C(O), CH₂ or CH-CH₃;
R6 is hydrogen, -(C₁-C₆)-alkyl, cyclopropyl or -(C₁-C₄)-alkylene-phenyl.

6. The compound of the formula I as claimed in any of claims 1 to 5, and/or all stereoisomeric or tautomeric forms of the compound of the formula I and/or mixtures of these forms in any ratio, and/or a physiologically acceptable salt of the compound of the formula I, where the compound of the formula I is selected from the group consisting of
4-{((E)-2-[5-(3-fluorophenyl)pyridin-2-yl]vinyl}-2-methyloctahydropyrrolo[3,4-a]-indolizine-1,3-dione,
4-[((E)-2-(5-bromopyridin-2-yl)vinyl]-2-methyloctahydropyrrolo[3,4-a]indolizine-1,3-dione,
7-hydroxy-2-methyl-4-[(E)-2-(6-methylpyridin-2-yl)vinyl]hexahydropyrrolo[3,4-a]-pyrrolizine-1,3-dione,
2-methyl-4-[(E)-2-(6-methylpyridin-2-yl)vinyl]octahydropyrrolo[3,4-a]indolizine-1,3-dione,
2-methyl-4-[(E)-2-(6-methylpyridin-2-yl)vinyl]hexahydropyrrolo[3,4-a]pyrrolizine-1,3-dione,
tert-butyl (8-{(E)-2-[5-(3-fluorophenyl)pyridin-2-yl]vinyl}-2-methyl-1,3-dioxodecahydro-2,5,7a-triazacyclopenta[a]indene-5-carboxylate,
(S)-4-{(E)-2-[5-(3-fluorophenyl)pyridin-2-yl]vinyl}-7-hydroxy-2-methylhexahydro-pyrrolo[3,4-a]pyrrolizine-1,3-dione,
(R)-4-{(E)-2-[5-(3-fluorophenyl)pyridin-2-yl]vinyl}-7-hydroxy-2-methylhexahydro-pyrrolo[3,4-a]pyrrolizine-1,3-dione,
6-{((E)-2-[5-(3-fluorophenyl)pyridin-2-yl]vinyl}-8-methylhexahydropyrrolo[3',4':3,4]-pyrrolo[2,1-c][1,4]oxazine-7,9-dione,
2-ethyl-4-{(E)-2-[5-(3-fluorophenyl)pyridin-2-yl]vinyl}octahydropyrrolo[3,4-a]indolizine-1,3-dione,
4-{((E)-2-[5-(3-fluorophenyl)pyridin-2-yl]vinyl}-2-methylhexahydropyrrolo[3,4-a]-pyrrolizine-1,3-dione,
5-{((E)-2-[5-(3-fluorophenyl)pyridin-2-yl]vinyl}-7-methyltetrahydropyrrolo[3',4':3,4]-pyrrolo[1,2-c]thiazole-6,8-dione,
2-methyl-4-{(E)-2-[5-(3-trifluoromethyl-phenyl)pyridin-2-yl]vinyl}octahydropyrrolo[3,4-a]indolizine-1,3-dione,
4-{((E)-2-[5-(2-methoxyphenyl)pyridin-2-yl]vinyl}-2-methyloctahydropyrrolo[3,4-a]-indolizine-1,3-dione,
4-{((E)-2-[5-(2-chlorophenyl)pyridin-2-yl]vinyl}-2-methyloctahydropyrrolo[3,4-a]-indolizine-1,3-dione,
2-methyl-4-[(E)-2-(5-thiophen-3-ylpyridin-2-yl)vinyl]octahydropyrrolo[3,4-a]indolizine-1,3-dione,
4-{((E)-2-[5-(3-fluorophenyl)pyridin-2-yl]vinyl}-2-methyl-3a,4,6,9,9a,9b-hexahydro-pyrrolo[3,4-a]indolizine-1,3-dione,
2-cyclopropyl-4-{(E)-2-[5-(3-fluorophenyl)pyridin-2-yl]vinyl}octahydropyrrolo[3,4-a]-indolizine-1,3-dione, and
8-{((E)-2-[5-(3-fluorophenyl)pyridin-2-yl]vinyl}-2-methyloctahydro-2,5,7a-triazacyclopenta[a]indene-1,3-dione.

7. A medicament having an effective content of at least one compound of the formula I or a physiologically acceptable salt thereof as claimed in one or more of claims 1 to 6 together with a pharmaceutically suitable and physiologically acceptable carrier, additive and/or other active compounds and auxiliaries.

8. The compound of the formula I or a physiologically acceptable salt thereof as claimed in one or more of claims 1 to 6 for use as pharmaceutic.

9. The compound of the formula I or a physiologically acceptable salt thereof as claimed in one or more of claims 1 to 6 for use for the prophylaxis, secondary prevention and therapy of all disorders associated with thromboses, embolisms, hypercoagulability, fibrotic changes or inflammatory disorders.

10. The compound of the formula I or a physiologically acceptable salt thereof as claimed in one or more of claims 1 to 6 for the use as claimed in claim 9, wherein the use is for myocardial infarction, angina pectoris and other types of acute coronary syndrome, acute stroke or its secondary prevention, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, reduction of the risk of thrombosis following surgical procedures such as knee and hip joint operations, procedures leading to contact of blood with foreign surfaces, such as for dialysis patients and patients with indwelling catheters, disseminated intravascular coagulation, sepsis and other intravascular events associated with inflammation, atherosclerosis, diabetes and the metabolic syndrome and the sequelae thereof, tumor growth and tumor metastasis, inflammatory and degenerative articular disorders such as rheumatoid arthritis and arthrosis, impairments of the hemostatic system such as fibrin deposits, fibrotic changes in the lung such as chronic obstructive pulmonary disease, adult respiratory distress syndrome, fibrin deposits in the eye following eye operations or prevention and/or treatment of scarring.

11. A process for preparing a compound of the formula I or a physiologically acceptable salt thereof as claimed in one or more of claims 1 to 6, which comprises
a) reacting a compound of the formula II, 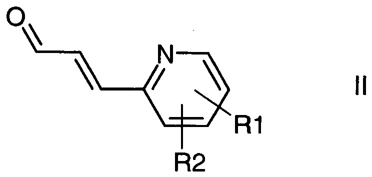 where the radicals R1 and R2 are as defined in formula I, with a compound of the formula III and a compound of the formula IV, 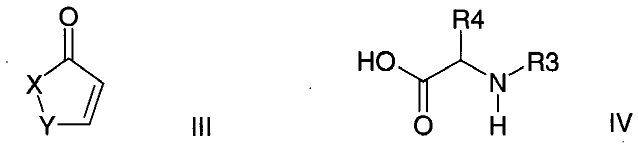 where the radicals X, Y, R3 and R4 are as defined in formula I, in the presence of a solvent at 20°C to 120°C to give a compound of the formula I; or
b) reacting a compound of the formula V, 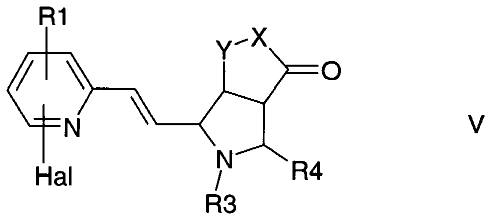 where the radicals X, Y, R1, R3 and R4 are as defined in formula I and Hal has the meaning of chlorine, bromine, iodine or triflate, with a compound of the formula R2-B(OH)₂ in the presence of a base and of a suitable metal catalyst in a suitable solvent or solvent mixture to give a compound of the formula I; or
c) reacting a compound of the formula I in which X has the meaning of NH with a suitable alkylating agent in the presence of a base and in a suitable inert solvent at room temperature or at elevated temperature to give the compound of the formula I in which X has the meaning of N-R6 and R6 is -(C₁-C₆)-alkyl, -(C₃-C₇)-cycloalkyl or -(C₁-C₄)-alkylene-aryl; or
d) fractionating the compound of the formula I which has been prepared by processes a) to c), or a suitable precursor of the formula I which, owing to its chemical structure, occurs in enantiomeric or diastereomeric forms, by salt formation with enantiomerically pure acids or bases, chromatography on chiral stationary phases or derivatization by means of chiral enantiomerically pure compounds such as amino acids, separation of the diastereomers obtained in this way, and elimination of the chiral auxiliary groups, into the pure enantiomers or diastereomers; or
e) either isolating the compound of the formula I prepared by processes a) to d) in free form or liberating it from non-physiologically acceptable salts or, in the case where acidic or basic groups are present, converting it into a physiologically acceptable salt.

## Revendications

1. Composé de formule I 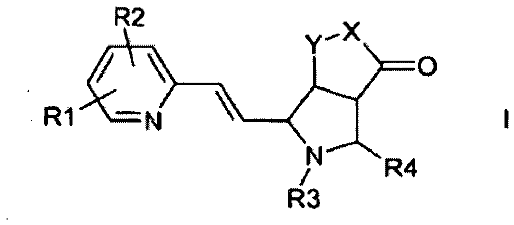 et/ou toutes les formes stéréoisomères ou tautomères du composé de formule I et/ou mélanges de ces formes en un rapport quelconque, et/ou sel physiologiquement compatible du composé de formule I, dans laquelle R1 et R2 sont identiques ou différents, et représentent indépendamment l'un de l'autre hydrogène, alkyle en (C₁-C₆), -O-alkyle en (C₁-C₆), aryle, halogène ou hétaryle ; alkyle, aryle et hétaryle étant chacun non substitués ou substitués une, deux ou trois fois indépendamment les unes des autres par F, Cl, Br, CN, alkyle en (C₁-C₄), -O-alkyle en (C₁-C₄), -NH-alkyle en (C₁-C₄),N(alkyle en (C₁-C₄))₂, -NH₂, -OH, -O-CF₃, -S-CF₃ ou -CF₃,
ou
R1 et R2 forment ensemble avec les atomes de cycle auxquels ils sont chacun reliés un cycle de 5 éléments à 6 éléments, le cycle n'étant constitué que d'atomes de carbone ou 1, 2 ou 3 de ces atomes étant remplacés par des atomes N, 0 ou S, le cycle étant non substitué ou substitué une, deux ou trois fois indépendamment les unes des autres par F, Cl, Br, CN, alkyle en (C₁-C₄),O-alkyle en (C₁-C₄), -NH-alkyle en (C₁-C₄), -N(alkyle en (C₁-C₄))₂, -NH₂, -OH, -O-CF₃, -S-CF₃ ou -CF₃ ;
R3 et R4 forment ensemble avec les atomes de cycle auxquels ils sont chacun reliés un cycle saturé ou insaturé de 5 éléments à 7 éléments, le cycle n'étant constitué que d'atomes de carbone ou 1 ou 2 de ces atomes étant remplacés par O, S, SO, SO₂ ou N-R5, le cycle étant non substitué ou substitué une, deux ou trois fois indépendamment les unes des autres par F, Cl, -OH, -NH-R7, alkyle en (C₁-C₄), -O-alkyle en (C₁-C₄), C(O)-O-alkyle en (C₁-C₄), aryle ou hétaryle ;
X représente N-R6 ou 0 ;
Y représente C(O), CH₂, CH-CH₃ ou C(CH₃)₂ ;
R5 représente hydrogène, -C(O)-alkyle en (C₁-C₄), -C(O)-O-alkyle en (C₁-C₄), alkyle en (C₁-C₆), alkyle en (C₂-C₄)-O-alkyle en (C₁-C₄), alkyle en (C₂-C₄)-OH, aryle ou hétaryle ;
R7 représente hydrogène, -C(O)-alkyle en (C₁-C₄), -C(O)-O-alkyle en (C₁-C₄), alkyle en (C₁-C₆), alkyle en (C₂-C₄)-O-alkyle en (C₁-C₄), alkyle en (C₂-C₄)-OH, aryle ou hétaryle ;
R6 représente hydrogène, alkyle en (C₁-C₆), cycloalkyle en (C₃-C₇), alkylène en (C₁-C₄)-aryle ou C(O)-O-alkyle en (C₁-C₄) ;
le terme « hétaryle » désignant des systèmes cycliques contenant 4 à 15 atomes de carbone, qui se présentent dans un, deux ou trois systèmes cycliques reliés les uns aux autres et qui contiennent, selon la taille des cycles, un, deux, trois ou quatre hétéroatomes identiques ou différents de la série constituée par oxygène, azote ou soufre.

2. Composé de formule I selon la revendication 1, et/ou toutes les formes stéréoisomères ou tautomères du composé de formule I et/ou mélanges de ces formes en un rapport quelconque, et/ou sel physiologiquement compatible du composé de formule I, dans laquelle R1 et R2 sont identiques ou différents, et représentent indépendamment l'un de l'autre hydrogène, alkyle en (C₁-C₆), -O-alkyle en (C₁-C₆), aryle, halogène ou hétaryle ; alkyle, aryle et hétaryle étant chacun non substitués ou substitués une, deux ou trois fois indépendamment les unes des autres par F, Cl, Br, CN, alkyle en (C₁-C₄), -O-alkyle en (C₁-C₄), -NH-alkyle en (C₁-C₄),N(alkyle en (C₁-C₄))₂ ou -CF₃, ou
R1 et R2 forment ensemble avec les atomes de cycle auxquels ils sont chacun reliés un cycle, le système cyclique bicyclique constitué de ce cycle et du cycle pyridine portant R1 et R2 étant choisi dans le groupe constitué par quinoline et isoquinoline ;
R3 et R4 forment ensemble avec les atomes de cycle auxquels ils sont chacun reliés un cycle saturé ou insaturé de 5 éléments à 7 éléments, le cycle n'étant constitué que d'atomes de carbone ou 1 ou 2 de ces atomes étant remplacés par 0, S, SO, SO₂ ou N-R5, choisi dans le groupe constitué par azépine, [1,2]diazépine, [1,3]diazépine, [1,4]diazépine, dihydroimidazolone, imidazole, imidazoline, imidazolidine, imidazolidinone, isothiazole, isothiazolidine, isothiazoline, isoxazole, isoxazoline, isoxazolidine, morpholine, dioxyde d'oxathiazine, [1,2]oxazine, [1,3]oxazine, [1,4]oxazine, oxazolone, oxazole, oxazolidine, pipérazine, pipéridine, pyrazine, pyrazole, pyrazoline, pyrazolidine, pyridazine, pyridine, pyridinone, pyridopyrazine, pyridopyridine, pyrimidine, pyrrole, pyrrolidine, pyrrolidinone, pyrroline, 1,2,3,6-tétrahydro-pyridine, [1,2]thiazine, [1,3]thiazine, [1,4]thiazine, [1,3]thiazole, thiazole ou thiazolidine, le cycle étant non substitué ou substitué une, deux ou trois fois indépendamment les unes des autres par F, Cl, -OH, alkyle en (C₁-C₄), -O-alkyle en (C₁-C₄), C(O)-O-alkyle en (C₁-C₄), aryle ou hétaryle ;
X représente N-R6 ou 0 ;
Y représente C(O), CH₂ ou CH-CH₃ ;
R5 représente hydrogène, C(O)-alkyle en (C₁-C₄), C(O)-O-alkyle en (C₁-C₄), alkyle en (C₁-C₆), aryle ou hétaryle ;
R6 représente hydrogène, alkyle en (C₁-C₆), cycloalkyle en (C₃-C₇) ou alkylène en (C₁-C₄)-aryle ;
le terme « hétaryle » désignant des systèmes cycliques contenant 4 à 15 atomes de carbone, qui se présentent dans un, deux ou trois systèmes cycliques reliés les uns aux autres et qui contiennent, selon la taille des cycles, un, deux, trois ou quatre hétéroatomes identiques ou différents de la série constituée par oxygène, azote ou soufre.

3. Composé de formule I selon les revendications 1 à 2, et/ou toutes les formes stéréoisomères ou tautomères du composé de formule I et/ou mélanges de ces formes en un rapport quelconque, et/ou sel physiologiquement compatible du composé de formule I, dans laquelle R1 et R2 sont identiques ou différents, et représentent indépendamment l'un de l'autre hydrogène, alkyle en (C₁-C₄), -O-alkyle en (C₁-C₄), Br, F, Cl, aryle ou hétaryle ; hétaryle étant choisi dans le groupe constitué par acridinyle, azépinyle, azétidinyle, aziridinyle, benzimidazolyle, benzofuranyle, benzothiofuranyle, benzothiophényle, benzoxazolyle, benzthiazolyle, benztriazolyle, benzisoxazolyle, benzisothiazolyle, carbazolyle, 4aH-carbazolyle, carbolinyle, quinazolinyle, quinolinyle, 4H-quinolizinyle, quinoxalinyle, quinuclidinyle, chromanyle, chroményle, cinnolinyle, décahydroquinolinyle, dibenzofuranyle, dibenzothiophényle, dihydrofurane[2,3-b]-tétrahydrofuranyle, dihydrofuranyle, dioxolyle, dioxanyle, 2H,6H-1,5,2-dithiazinyle, furanyle, furazanyle, imidazolidinyle, imidazolinyle, imidazolyle, 1H-indazolyle, indolinyle, indolizinyle, indolyle, 3H-indolyle, isobenzofuranyle, isoquinolinyle, isochromanyle, isoindazolyle, isoindolinyle, isoindolyle, isothiazolidinyle, 2-isothiazolinyle, isothiazolyle, isoxazolyle, isoxazolidinyle, 2-isoxazolinyle, morpholinyle, naphtyridinyle, octahydroisoquinolinyle, 1,2,3-oxadiazolyle, 1,2,4-oxadiazolyle, 1,2,5-oxadiazolyle, 1,3,4-oxadiazolyle, oxazolidinyle, oxazolyle, oxothiolanyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purinyle, pyranyle, pyrazinyle, pyrazolidinyle, pyrazolinyle, pyrazolyle, pyridazinyle, pyridooxazolyle, pyridoimidazolyle, pyridothiazolyle, pyridothiophényle, pyridyle, pyrimidinyle, pyrrolidinyle, pyrrolinyle, 2H-pyrrolyle, pyrrolyle, tétrahydrofuranyle, tétrahydroisoquinolinyle, tétrahydroquinolinyle, tétrahydropyridinyle, 6H-1,2,5-thiadiazinyle, 1,2,3-thiadiazolyle, 1,2,4-thiadiazolyle, 1,2,5-thiadiazolyle, 1,3,4-thiadiazolyle, thianthrényle, thiazolidinyle, thiazolinyle, thiazolyle, thiényle, thiénoimidazolyle, thiénooxazolyle, thiénopyrrolyle, thiénopyridinyle, thiénothiazolyle, thiénothiophényle, thiomorpholinyle, triazinyle, 1,2,3-triazolyle, 1,2,4-triazolyle, 1,2,5-triazolyle, 1,3,4-triazolyle et xanthényle ; alkyle, aryle et hétaryle étant chacun non substitués ou substitués une, deux ou trois fois indépendamment les unes des autres par F, Cl, CN, alkyle en (C₁-C₄), -O-alkyle en (C₁-C₄), -N(alkyle en (C₁-C₄))₂ ou -CF₃ ;
R3 et R4 forment ensemble avec les atomes de cycle auxquels ils sont chacun reliés un cycle saturé ou insaturé de 5 éléments à 7 éléments, choisi dans le groupe constitué par azépine, [1,2]diazépine, [1,3]diazépine, [1,4]diazépine, dihydroimidazolone, imidazole, imidazoline, imidazolidine, imidazolidinone, isothiazole, isothiazolidine, isothiazoline, isoxazole, isoxazoline, isoxazolidine, morpholine, dioxyde d'oxathiazine, [1,2]oxazine, [1,3]oxazine, [1,4]oxazine, oxazolone, oxazole, oxazolidine, pipérazine, pipéridine, pyrazine, pyrazole, pyrazoline, pyrazolidine, pyridazine, pyridine, pyridinone, pyrimidine, pyrrole, pyrrolidine, pyrrolidinone, pyrroline, 1,2,3,6-tétrahydro-pyridine, [1,2]thiazine, [1,3]thiazine, [1,4]thiazine, [1,3]thiazole, thiazole et thiazolidine, le cycle étant non substitué ou substitué une, deux ou trois fois indépendamment les unes des autres par F, Cl, -OH, alkyle en (C₁-C₄), C(O)-O-alkyle en (C₁-C₄), aryle ou hétaryle ;
X représente N-R6 ou 0 ;
Y représente C(O), CH₂ ou CH-CH₃ ;
R6 représente hydrogène, alkyle en (C₁-C₆), cycloalkyle en (C₃-C₆) ou alkylène en (C₁-C₄)-aryle.

4. Composé de formule I selon les revendications 1 à 3, et/ou toutes les formes stéréoisomères ou tautomères du composé de formule I et/ou mélanges de ces formes en un rapport quelconque, et/ou sel physiologiquement compatible du composé de formule I, dans laquelle R1 et R2 sont identiques ou différents, et représentent indépendamment l'un de l'autre hydrogène, alkyle en (C₁-C₄), Br, thiényle ou phényle ; thiényle et phényle étant chacun non substitués ou substitués une ou deux fois indépendamment l'une de l'autre par F, Cl, -O-méthyle ou -CF₃ ;
R3 et R4 forment ensemble avec les atomes de cycle auxquels ils sont chacun reliés un cycle saturé ou insaturé de 5 éléments à 6 éléments, choisi dans le groupe constitué par azépine, [1,2]diazépine, [1,3]diazépine, [1,4]diazépine, dihydroimidazolone, imidazole, imidazoline, imidazolidine, imidazolidinone, isothiazole, isothiazolidine, isothiazoline, isoxazole, isoxazoline, isoxazolidine, morpholine, dioxyde d'oxathiazine, [1,2]oxazine, [1,3]oxazine, [1,4]oxazine, oxazolone, oxazole, oxazolidine, pipérazine, pipéridine, pyrazine, pyrazole, pyrazoline, pyrazolidine, pyridazine, pyridine, pyridinone, pyrimidine, pyrrole, pyrrolidine, pyrrolidinone, pyrroline, 1,2,3,6-tétrahydro-pyridine, [1,2]thiazine, [1,3]thiazine, [1,4]thiazine, [1,3]thiazole, thiazole et thiazolidine, le cycle étant non substitué ou substitué une, deux ou trois fois indépendamment les unes des autres par F, -OH, C(O)-O-alkyle en (C₁-C₄),
phényle ou hétaryle ;
X représente N-R6 ou O ;
Y représente C(O), CH₂ ou CH-CH₃ ;
R6 représente hydrogène, alkyle en (C₁-C₆), cycloalkyle en (C₃-C₆) ou alkylène en (C₁-C₄)-phényle.

5. Composé de formule I selon les revendications 1 à 4, et/ou toutes les formes stéréoisomères ou tautomères du composé de formule I et/ou mélanges de ces formes en un rapport quelconque, et/ou sel physiologiquement compatible du composé de formule I, dans laquelle R1 et R2 sont identiques ou différents, et représentent indépendamment l'un de l'autre hydrogène, alkyle en (C₁-C₄), Br, thiényle ou phényle ; thiényle et phényle étant chacun non substitués ou substitués une ou deux fois indépendamment l'une de l'autre par F, Cl, -O-méthyle ou -CF₃ ;
R3 et R4 forment ensemble avec les atomes de cycle auxquels ils sont chacun reliés un cycle saturé ou insaturé de 5 éléments à 6 éléments, choisi dans le groupe constitué par morpholine, pipérazine, pipéridine, pyrrolidine, 1,2,3,6-tétrahydro-pyridine ou [1,3]thiazole, le cycle étant non substitué ou substitué une, deux ou trois fois indépendamment les unes des autres par F, -OH, phényle ou C(O)-O-alkyle en (C₁-C₄) ;
X représente N-R6 ;
Y représente C(O), CH₂ ou CH-CH₃ ;
R6 représente hydrogène, alkyle en (C₁-C₆), cyclopropyle ou alkylène en (C₁-C₄)-phényle.

6. Composé de formule I selon les revendications 1 à 5, et/ou toutes les formes stéréoisomères ou tautomères du composé de formule I et/ou mélanges de ces formes en un rapport quelconque, et/ou sel physiologiquement compatible du composé de formule I, le composé de formule I étant choisi dans le groupe constitué par :
la 4-{(E)-2-[5-(3-fluoro-phényl)-pyridin-2-yl]-vinyl}-2-méthyl-octahydro-pyrrolo[3,4-a]indolizine-1,3-dione, la 4-[(E)-2-(5-bromo-pyridin-2-yl)-vinyl]-2-méthyl-octahydro-pyrrolo[3,4-a]indolizine-1,3-dione,
la 7-hydroxy-2-méthyl-4-[(E)-2-(6-méthyl-pyridin-2-yl)-vinyl]-hexahydro-pyrrolo[3,4-a]-pyrrolizine-1,3-dione,
la 2-méthyl-4-[(E)-2-(6-méthyl-pyridin-2-yl)-vinyl]-octahydro-pyrrolo[3,4-a]-indolizine-1,3-dione,
la 2-méthyl-4-[(E)-2-(6-méthyl-pyridin-2-yl)-vinyl]-hexahydro-pyrrolo[3,4-a]-pyrrolizine-1,3-dione,
l'ester tert-butylique de l'acide (8-{(E)-2-[5-(3-fluoro-phényl)-pyridin-2-yl]-vinyl}-2-méthyl-1,3-dioxo-décahydro-2,5,7a-triazacyclopenta[a]indène-5-carboxylique,
la (S)-4-{(E)-2-[5-(3-fluoro-phényl)-pyridin-2-yl]-vinyl}-7-hydroxy-2-méthyl-hexahydro-pyrrolo[3,4-a]pyrrolizine-1,3-dione,
la (R)-4-{(E)-2-[5-(3-fluoro-phényl)-pyridin-2-yl]-vinyl}-7-hydroxy-2-méthyl-hexahydro-pyrrolo[3,4-a]pyrrolizine-1,3-dione,
la 6-{(E)-2-[5-(3-fluoro-phényl)-pyridin-2-yl]-vinyl}-8-méthyl-hexahydro-pyrrolo[3',4':3,4]pyrrolo[2,1-c][1,4]oxazine-7,9-dione,
la 2-éthyl-4-{(E)-2-[5-(3-fluoro-phényl)-pyridin-2-yl]-vinyl}-octahydro-pyrrolo[3,4-a]indolizine-1,3-dione,
la 4-{(E)-2-[5-(3-fluoro-phényl)-pyridin-2-yl]-vinyl}-2-méthyl-hexahydro-pyrrolo[3,4-a]pyrrolizin-1,3-dione,
la 5-{(E)-2-[5-(3-fluoro-phényl)-pyridin-2-yl]-vinyl}-7-méthyl-tétrahydro-pyrrolo[3',4':3,4]pyrrolo[1,2-c]thiazole-6,8-dione,
la 2-méthyl-4-{(E)-2-[5-(3-trifluorométhyl-phényl)-pyridin-2-yl]-vinyl}-octahydro-pyrrolo[3,4-a]indolizine-1,3-dione,
la 4-{(E)-2-[5-(2-méthoxy-phényl)-pyridin-2-yl]-vinyl}-2-méthyl-octahydro-pyrrolo[3,4-a]indolizine-1,3-dione,
la 4-{(E)-2-[5-(2-chloro-phényl)-pyridin-2-yl]-vinyl}-2-méthyl-octahydro-pyrrolo[3,4-a]indolizine-1,3-dione,
la 2-méthyl-4-[(E)-2-(5-thiophén-3-yl-pyridin-2-yl)-vinyl]-octahydro-pyrrolo[3,4-a]indolizine-1,3-dione,
la 4-{(E)-2-[5-(3-fluoro-phényl)-pyridin-2-yl]-vinyl}-2-méthyl-3a,4,6,9,9a,9b-hexahydro-pyrrolo[3,4-a]indolizine-1,3-dione,
la 2-cyclopropyl-4-{(E)-2-[5-(3-fluoro-phényl)-pyridin-2-yl]-vinyl}-octahydro-pyrrolo[3,4-a]indolizine-1,3-dione et
la 8-{(E)-2-[5-(3-fluoro-phényl)-pyridin-2-yl]-vinyl}-2-méthyl-octahydro-2,5,7a-triazacyclopenta[a]indène-1,3-dione.

7. Médicament, **caractérisé par** une teneur efficace en au moins un composé de formule I ou un sel physiologiquement compatible de celui-ci selon une ou plusieurs des revendications 1 à 6, conjointement avec un véhicule pharmaceutiquement approprié et physiologiquement compatible, un additif et/ou d'autres agents actifs et adjuvants.

8. Composé de formule I ou sel physiologiquement compatible de celui-ci selon une ou plusieurs des revendications 1 à 6, destiné à une utilisation en tant que produit pharmaceutique.

9. Composé de formule I ou sel physiologiquement compatible de celui-ci selon une ou plusieurs des revendications 1 à 6, destiné à une utilisation pour la prophylaxie, la prévention secondaire et le traitement de toutes les maladies qui accompagnent les thromboses, les embolies, l'hypercoagulabilité, les modifications fibrotiques ou les maladies inflammatoires.

10. Composé de formule I ou sel physiologiquement compatible de celui-ci selon une ou plusieurs des revendications 1 à 6, destiné à une utilisation selon la revendication 9, **caractérisé en ce qu'**il s'agit d'un infarctus du myocarde, d'une angine de poitrine et d'autres formes du syndrome coronarien aigu, de l'attaque cérébrale aigue ou de sa prévention secondaire, des maladies vasculaires périphériques, de la thrombose veineuse profonde, de l'embolie pulmonaire, des événements emboliques ou thrombotiques causés par des arythmies cardiaques, des évènements cardiovasculaires tels que les resténoses après une revascularisation et une angioplastie et des interventions semblables telles que des implantations de stents et des opérations de pontage, de la réduction du risque de thrombose après des interventions chirurgicales telles que des opérations du genou et des opérations de la hanche, des interventions qui conduisent à un contact du sang avec des surfaces étrangères telles que pour les patients recevant une dialyse et les patients munis de sondes à demeure, de la coagulation intravasculaire disséminée, du sepsis et d'autres événements intravasculaires qui accompagnent une inflammation, de l'athérosclérose, du diabète et du syndrome métabolique et de leurs conséquences, de la croissance de tumeurs et de la métastase de tumeurs, des maladies inflammatoires et dégénératives des articulations, telles que la polyarthrite rhumatoïde et l'arthrose, des troubles du système hémostatique tels que les dépôts de fibrine, des modifications fibrotiques des poumons, telles que la pneumopathie obstructive chronique, du syndrome de détresse respiratoire de l'adulte, des dépôts de fibrine de l'oeil après des opérations de l'oeil, ou de la prévention et/ou du traitement de la formation de cicatrices.

11. Procédé de fabrication d'un composé de formule I ou d'un sel physiologiquement compatible de celui-ci selon une ou plusieurs des revendications 1 à 6, **caractérisé en ce que**
a) un composé de formule II 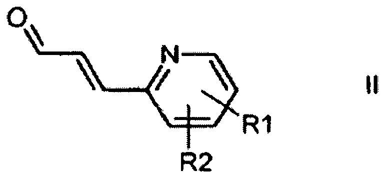 dans laquelle les radicaux R1 et R2 sont tels que définis pour la formule I, est mis en réaction avec un composé de formule III et un composé de formule IV 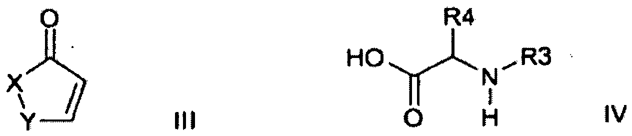 dans lesquelles les radicaux X, Y, R3 et R4 sont tels que définis pour la formule I, en présence d'un solvant, à une température de 20 °C à 120 °C, pour former un composé de formule I ; ou
b) un composé de formule V 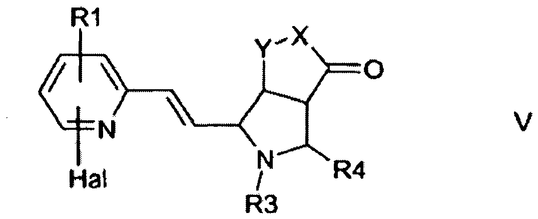 dans laquelle les radicaux X, Y, R1, R3 et R4 sont tels que définis pour la formule I, et Hal a la signification chlore, brome, iode ou triflate, est mis en réaction avec un composé de formule R2-B(OH)₂ en présence d'une base et d'un catalyseur métallique approprié dans un solvant ou mélange de solvants approprié pour former un composé de formule I ; ou
c) un composé de formule I, dans laquelle X a la signification NH, est mis en réaction avec un agent d'alkylation approprié en présence d'une base et dans un solvant inerte approprié, à température ambiante ou à une température élevée, pour former le composé de formule I, dans laquelle X a la signification N-R6 et R6 signifie alkyle en (C₁-C₆), cycloalkyle en (C₃-C₇) ou alkylène en (C₁-C₄)-aryle ; ou
d) le composé de formule I fabriqué par les procédés a) à c), ou un précurseur approprié de la formule I, qui se présente en raison de sa structure chimique sous des formes énantiomères ou diastéréomères, est séparé par formation d'un sel avec des acides ou bases énantiomériquement purs, chromatographie sur des phases stationnaires chirales ou dérivation avec des composés chiraux énantiomériquement purs tels que des acides aminés, séparation des diastéréomères ainsi obtenus et clivage des groupes auxiliaires chiraux en les énantiomères ou diastéréomères purs ; ou
e) le composé de formule I fabriqué par les procédés a) à d) est isolé sous forme libre ou libéré de sels physiologiquement incompatibles ou, lors de la présence de groupes acides ou basiques, transformé en un sel physiologiquement compatible.
